(19) Europäisches Patentamt / European Patent Office / Office européen des brevets

(11) **EP 3 298 405 B1**

(12) **EUROPEAN PATENT SPECIFICATION**

(45) Date of publication and mention
of the grant of the patent:
**09.10.2019 Bulletin 2019/41**

(21) Application number: **16727297.0**

(22) Date of filing: **20.05.2016**

(51) Int Cl.:
**G01N 33/53** (2006.01)

(86) International application number:
**PCT/DK2016/050138**

(87) International publication number:
**WO 2016/188530 (01.12.2016 Gazette 2016/48)**

(54) **BIOMARKERS FOR PREDICTION OF DEVELOPMENT OF HYPOXEMIA DUE TO ACUTE LUNG INJURY**

BIOMARKER ZUR VORHERSAGE DER ENTWICKLUNG VON HYPOXEMIA DURCH AKUTE LUNGENVERLETZUNGEN

BIOMARQUEURS POUR LA PRÉDICTION DU DÉVELOPPEMENT D'UNE HYPOXÉMIE DUE À UNE LÉSION PULMONAIRE AIGUË

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2015 EP 15168879**

(43) Date of publication of application:
**28.03.2018 Bulletin 2018/13**

(73) Proprietors:
• **Region Nordjylland, Aalborg Sygehus
9220 Aalborg Ø (DK)**
• **Aalborg Universitet
9220 Aalborg Ø (DK)**

(72) Inventors:
• **MALTESEN, Raluca Georgiana
9000 Aalborg (DK)**
• **RASMUSSEN, Bodil Steen
9000 Aalborg (DK)**
• **WIMMER, Reinhard
9230 Svenstrup J. (DK)**
• **PEDERSEN, Shona
9220 Aalborg Ø (DK)**
• **KRISTENSEN, Søren Risom
9000 Aalborg (DK)**
• **HANIFA, Munsoor Ali
9400 Nørresundby (DK)**

(74) Representative: **Plougmann Vingtoft a/s
Strandvejen 70
2900 Hellerup (DK)**

(56) References cited:
• **BARBARA BOJKO ET AL: "Metabolic profiling of plasma from cardiac surgical patients concurrently administered with tranexamic acid: DI-SPME-LC-MS analysis", JOURNAL OF PHARMACEUTICAL ANALYSIS, vol. 4, no. 1, 1 February 2014 (2014-02-01), pages 6-13, XP055214280, ISSN: 2095-1779, DOI: 10.1016/j.jpha.2013.03.002**
• **NATALIE J. SERKOVA ET AL: "The Emerging Field of Quantitative Blood Metabolomics for Biomarker Discovery in Critical Illnesses", AMERICAN JOURNAL OF RESPIRATORY AND CRITICAL CARE MEDICINE, vol. 184, no. 6, 15 September 2011 (2011-09-15), pages 647-655, XP055214610, ISSN: 1073-449X, DOI: 10.1164/rccm.201103-0474CI**
• **K. A. STRINGER ET AL: "Metabolic consequences of sepsis-induced acute lung injury revealed by plasma 1H-nuclear magnetic resonance quantitative metabolomics and computational analysis", AMERICAN JOURNAL OF PHYSIOLOGY. LUNG CELLULAR AND MOLECULAR PHYSIOLOGY, vol. 300, no. 1, 1 October 2010 (2010-10-01), pages 4-11, XP055214614, ISSN: 1040-0605, DOI: 10.1152/ajplung.00231.2010**

- ASAD A SHAH ET AL: "Metabolic profiles predict adverse events after coronary artery bypass grafting", JOURNAL OF THORACIC AND CARDIOVASCULAR SURGERY, MOSBY-YEAR BOOK, INC., ST. LOUIS, MO, US, vol. 143, no. 4, 15 September 2011 (2011-09-15), pages 873-878, XP028468584, ISSN: 0022-5223, DOI: 10.1016/J.JTCVS.2011.09.070 [retrieved on 2012-01-10]
- P. AGOSTONI ET AL: "Surfactant protein B and RAGE increases in the plasma during cardiopulmonary bypass: a pilot study", EUROPEAN RESPIRATORY JOURNAL, vol. 37, no. 4, 22 July 2010 (2010-07-22), pages 841-847, XP055214072, ISSN: 0903-1936, DOI: 10.1183/09031936.00045910
- ENGELS GERWIN E ET AL: "The effect of pulsatile cardiopulmonary bypass on lung function in elderly patients", INTERNATIONAL JOURNAL OF ARTIFICIAL ORGANS, WICHTIG PUBLISHING, IT, vol. 37, no. 9, 1 September 2014 (2014-09-01), pages 679-687, XP009186093, ISSN: 0391-3988
- GERWIN E ENGELS ET AL: "The utility of lung epithelium specific biomarkers in cardiac surgery: a comparison of biomarker profiles in on- and off-pump coronary bypass surgery", JOURNAL OF CARDIOTHORACIC SURGERY, BIOMED CENTRAL LTD, LO, vol. 8, no. 1, 9 January 2013 (2013-01-09), page 4, XP021137505, ISSN: 1749-8090, DOI: 10.1186/1749-8090-8-4
- GERWIN ERIK ENGELS ET AL: "Biomarkers of Lung Injury in Cardiothoracic Surgery", DISEASE MARKERS, vol. 18, no. 3, 1 January 2015 (2015-01-01), pages 203-10, XP055213330, ISSN: 0278-0240, DOI: 10.1371/journal.pone.0111466

**Description**

**Technical field of the invention**

The present invention relates to biomarkers for early detection of hypoxemia caused by acute lung injury. In particular, the present invention relates to biomarkers for early detection of patients in risk of hypoxemia who have undergone open cardiac surgery with the use of cardiopulmonary bypass (CPB).

**Background of the invention**

Acute lung injury is today diagnosed by the degree of hypoxemia and the condition is life-threatening, affecting more than one million individuals worldwide every year. The condition can develop within a week after pathophysiological events such as pneumonia, inhalation of toxic agent, aspiration of gastric content, sepsis, CPB, major surgery, blood transfusion and severe trauma among others. Regardless of the initial triggering factor, the result is abnormal gas exchange caused by excessive inflammation and disordered coagulation. Endothelial cell swelling, cellular junction widening, oedema accumulation, denuded alveolar membrane, increased pinocytosis, and alveolar collapse are the hallmarks of this dysfunction.

Currently, no therapeutic intervention has proven useful in impeding disease progression and no 'gold standard' diagnostic test exists. Therefore, diagnosis is presently based on four criteria: acute onset of symptoms, arterial hypoxemia, bilateral lung infiltrates on thoracic x-ray, and absence of left arterial hypertension. The arterial hypoxemia occur relatively late and are thus a poor predictor for early diagnosis. As such, identifying patients at risk of developing acute lung injury earlier in the course of their illness would enable the development of therapeutic options targeting the disease.

Substantial efforts have been made to understand acute lung injury and multiple mechanisms appear to be involved. In general, the research is focused on understanding the delicate balance between protective and injurious immunologic responses.

Bojko et al. (Journal of Pharmaceutical Analysis, vol. 4, no. 1, 1 February 2014 (2014-02-01), pages 6-13) discloses metabolic profiles of plasma samples from patients undergoing heart surgery with the use of CPB. Bojko et al. is silent in respect of hypoxaemia or acute lung injury.

Serkova et al. ((2011), American Journal of respiratory and Critical Care Medicine, vol. 184, no. 6, pages 647-655) and Stringer et al. (American Journal of Physiology. Lung Cellular and Molecular Physiology, vol. 300, no. 1, 1 October 2010 (2010-10-01), pages 4-11) relate to blood metabolomics for biomarker discovery in critical illnesses, such as in patients having sepsis-induced ALI. Both documents disclose a system comprising an analyser and processor for performing metabolomics.

Shah et al. (Journal of Thoracic and Cardiovascular Surgery),vol. 143, no. 4, (2011-09-15), pages 873-878) discloses metabolic profiles of short-chain dicarboxylacylcarnitines, ketone-related metabolites,and short-chain acylcarnitines which are associated with an adverse outcome after coronary artery bypass grafting (CABG). Adverse outcomes are considered to be event free survival, myocardial infarction, percutaneous coronary intervention or a repetition of CABG. There is however no sampling done during or shortly after surgery.

Agostoni et al., (European Respiratory Journal, vol. 37, no. 4, 22 July 2010 (2010-07-22), pages 841-847) discloses that Surfactant protein B and RAGE increase in the plasma during cardiopulmonary bypass. Haemodynamic and gas exchange parameters are recorded during surgery and in the 2 following days.

Engels et al. (International Journal of Artificial Organs, vol. 37, no. 9, (2014-09-01), pages 679-687) discloses the use of Elastase, CC16 and surfactant protein D as biomarkers for checking lung function in patients after CPB. Blood samples were taken before the operation, at wound closure, 2 and 18 hours after arrival at the intensive care unit. PaO2/FiO2 and Aa-02 gradient are also measured. No measurement of the metabolites was performed.

Engels et al. (Journal of Cardiothoracic Surgery, vol. 8, no. 1, 9 January 2013 (2013-01-09), page 4) discloses that SPD and CC16 are used as biomarkers in cardiac surgery. Lung dysfunction is also measured but no measurement of the metabolites.

Engels et al. (Disease Markers, vol. 18, no. 3, 1 January 2015 (2015-01-01), pages 203-210) is a review on biomarkers (peptides, no metabolites) of Lung Injury in Cardiothoracic Surgery.

Hence, an improved method for identifying patients at risk of developing hypoxemic acute lung injury would be advantageous, and in particular a more efficient and/or reliable method of identifying patients in risk of developing hypoxemia would be beneficial for clinicians and patient-care.

**Summary of the invention**

Pulmonary dysfunction remains the most common complication of cardiac surgery. The present invention relates to a set of biomarkers, which may be used to predict patients will develop hypoxemia after open cardiac surgery with the use of cardiopulmonary bypass (CPB). It was hypothesized that early stages of postoperative acute lung injury may be reflected at the metabolite level. It was therefore investigated whether metabolite changes in blood collected from the pulmonary artery (PA) and left atrium (LA) on the first postoperative day could function as biomarkers for the development of hypoxemia. Remarkably, powerful predictive biomarkers of postoperative acute lung injury were identified two days prior to clinical diagnosis.

Thus, an object of the present invention relates to the identification of predictive biomarkers for the development of hypoxemia.

Thus, one aspect of the invention relates to a method for determining the risk of developing hypoxemia, the method comprising

- providing a biological sample (said sample has been provided) from a subject, said subject having had open cardiac surgery with the use of cardiopulmonary bypass (CPB), wherein said sample has been provided from said subject between start of surgery (defined as time of skin incision) and 72 hours after detachment from the CPB circuit;

- determining the level of at least one biomarker selected from the group consisting of carnitine, citrate, isobutyrylglycine, O-N-Acetyl-Glucosamine, arachidonic acid, and isobutyric acid;
  and/or

- determining the ratio between levels of biomarkers of at least one ratio selected from citrate/phenylalanine, PUFA/phenylalanine, alanine/phenylalanine, citrate/Trimethylamine-N-oxide (TMAO), carnitine/TMAO, isobutyric acid/phenylalanine, and/or isobutyrylglycine/arginine;

- comparing said levels of one or more biomarkers or ratios to one or more reference (metabolic) levels;

- determining that said subject is at risk of developing hypoxemia if said level of one or more biomarkers are above the one or more reference levels, or determining that said subject is not at risk of developing hypoxemia, if said one or more levels are equal to or below the one or more reference levels;
  and/or

- determining that said subject is at risk of developing hypoxemia if said one or more ratios between levels of biomarkers are above the one or more reference levels, or determining that said subject is not at risk of developing hypoxemia, if said level of one or more ratios between levels of biomarkers are equal to or below the one or more reference levels.

A second aspect of the invention relates to a method for determining the risk of developing hypoxemia, the method comprising

- providing a biological sample, such as serum, plasma, or full blod (said sample has been provided) from a subject, said subject having had open cardiac surgery with the use of cardiopulmonary bypass (CPB), wherein said sample has been provided from said subject between the time of weaning from the CPB (0 hours) (or start of surgery (defined as time of skin incision)) and 20 hours after detachment/weaning from the CPB circuit;

- determining the level of at least one biomarker selected from the group consisting of Trigonelline; Hypoxanthine; Dopamine; Dihydroxyphenylalanine (Dopa); and Inosine;

- comparing said levels of one or more biomarkers to one or more reference (metabolic) levels;

- determining that said subject is at risk of developing hypoxemia if said level of at least one of the metabolites Trigonelline, Hypoxanthine, Dopamine, and Inosine are below the one or more reference levels, and/or determining that said subject is at risk of developing hypoxemia, if said level of Dihydroxyphenylalanine (Dopa) is above the one or more reference levels;
  or

- determining that said subject is not at risk of developing hypoxemia if said level of at least one of the metabolites Trigonelline, Hypoxanthine, Dopamine, and Inosine are above or equal to the one or more reference levels, and/or determining that said subject is not at risk of developing hypoxemia, if said level of Dihydroxyphenylalanine (Dopa) is below or equal to the one or more reference levels;

Another aspect of the present invention relates to a method for evaluating the efficacy of treatment to prevent hypoxemia, the method comprising

- completing the method according to the first (and/or second) aspect of the invention;

- for a subject considered at risk of developing hypoxemia, evaluating whether a treatment protocol for the prevention of hypoxemia results in said subject not developing hypoxemia within a week from detachment from the CPB circuit.

A further aspect of the present invention relates to a system arranged for determining the risk of developing hypoxemia; the system comprising

- an analyser, such as an NMR spectrometer or a mass spectrometer, being arranged

    - to receive a biological sample from a subject, said subject having had open cardiac surgery with the use of cardiopulmonary bypass (CPB), wherein said sample has been provided from said subjectbetween start of surgery (defined as skin incision) and 72 hours after detachment from the CPB circuit; and

    - to determine the level of at least one biomarker selected from the group consisting of carnitine, isobutyrylglycine, N-Acetyl-Glucosamine, arachidonic and eicosapentanoic acid, isobutyric acid and citrate;
    and/or

    - determining the ratio between levels of biomarkers of at least one ratio selected from the group of ratios selected from citrate/phenylalanine, PUFA/phenylalanine, alanine/phenylalanine, citrate/ TMAO, carnitine/TMAO, isobutyric acid/phenylalanine, and isobutyrylglycine/arginine;

- a processor arranged

- to compare said levels of one or more biomarkers or ratios to one or more reference levels; and

- to determine that said subject is at risk of developing hypoxemia if said level of one or more biomarkers are above the one or more reference levels, or determining that said subject is not at risk of developing hypoxemia, if said one or more levels are equal to or below the one or more reference levels;
and/or

- to determine that said subject is at risk of developing hypoxemia if said one or more ratios between levels of biomarkers are above the one or more reference levels, or determining that said subject is not at risk of developing hypoxemia, if said level of one or more ratios between levels of biomarkers are equal to or below the one or more reference levels.

It is to be understood that such system may be combined by an existing analyser, e.g. an NMR spectrometer or a mass spectrometer, and a computer, e.g. a general computer provided with dedicated software arranged to perform at least part of one of the above-mentioned methods of the present invention.

**Brief description of the figures**

*Figure 1*

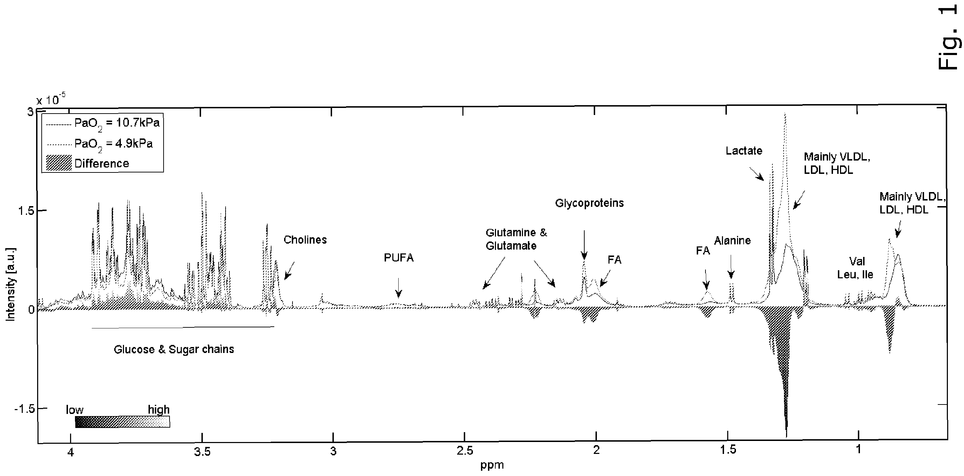

Figure 1 shows serum metabolic fingerprints: Representative 600 MHz one-dimensional Carr-Purcell-Meiboom-Gill (CPMG) 1H-nuclear magnetic resonance (NMR) spectral profiles of a patient presenting no sign of pulmonary dysfunction nor hypoxemia (partial pressure of oxygen in arterial blood, $PaO_2$= 10.7kPa or 80.3mmHg) (black) and a patient diagnosed with acute lung injury defined by hypoxemia ($PaO_2$=4.9kPa or 36.8mmHg) (grey) 72 hours after weaning from cardiopulmonary bypass (CPB). Spectrum difference (unaffected - hypoxemia) reveals metabolite fluctuations between patients. Higher levels of signals from lipoproteins, fatty acids and lower levels of some smaller metabolites are observed in the hypoxemic compared to unaffected patient.

*Figure 2*

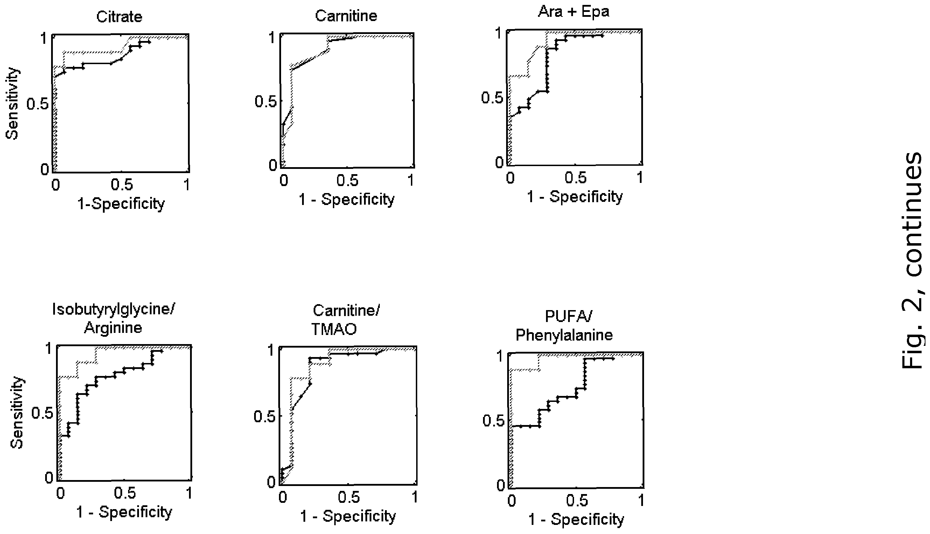

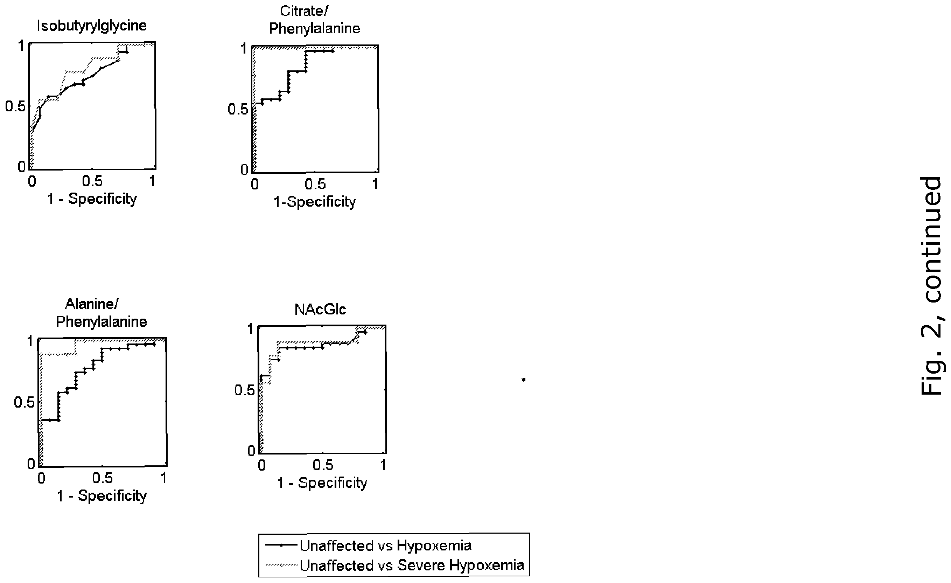

Figure 2 shows the Receiver Operating Characteristic (ROC) curve of biomarkers of early progression into acute lung injury after cardiac surgery. Biomarker levels (either concentrations of single metabolites or ratios between two metabolites) were measured on the first morning after weaning from CPB.

*Figure 3*

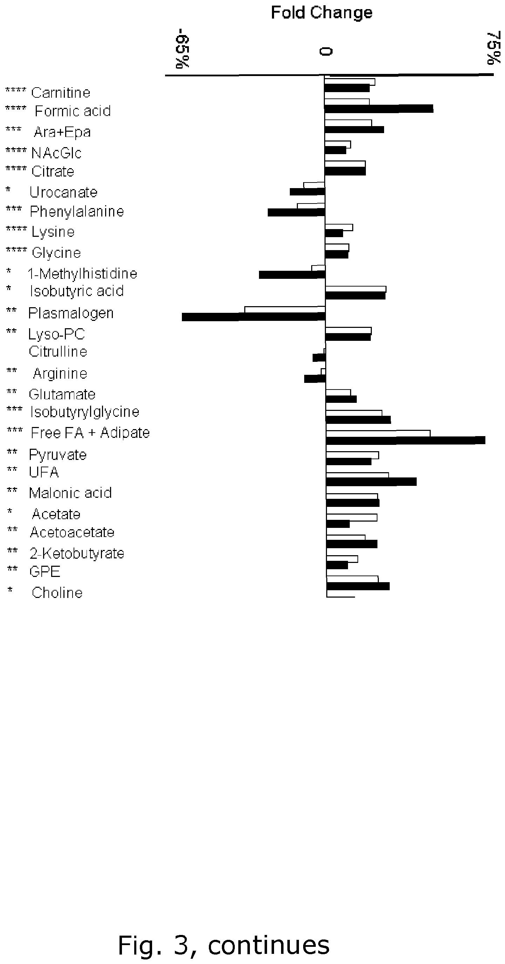

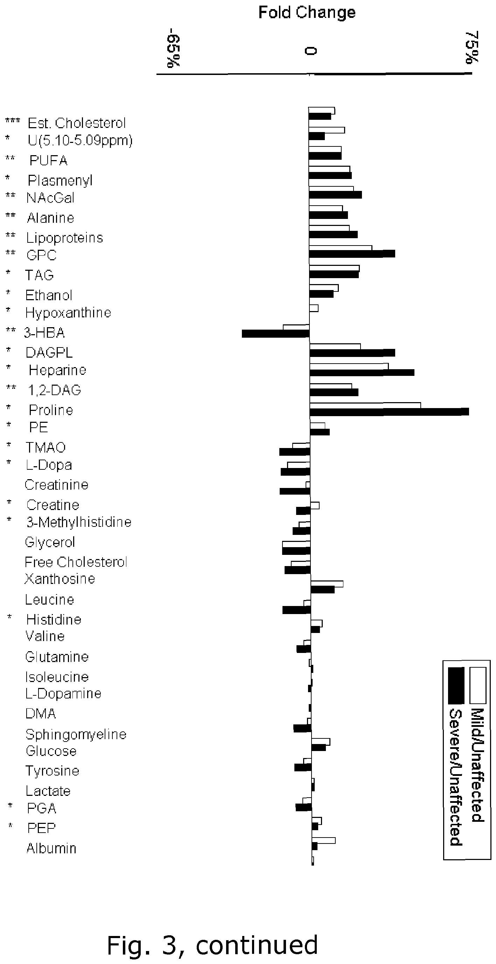

Figure 3 shows differences in metabolite levels between patient groups expressed as "fold-changes". Metabolic fold changes were calculated as mean percent change between mild vs. unaffected patients (open rectangles) and severe vs. unaffected patients (filled rectangles) ((A-U)/U)*100), where A= affected (mild or severe) and U= unaffected). Metabolites labelled with an asterisk were found to significantly vary between groups by using one-way ANOVA or its nonparametric analogue Kruskal-Wallis test ($*0.09 > p \geq 0.01$, $**10^{-2} > p \geq 10^{-3}$, $***10^{-4} > p \geq 10^{-5}$, $****p < 10^{-5}$).

*Figure 4*

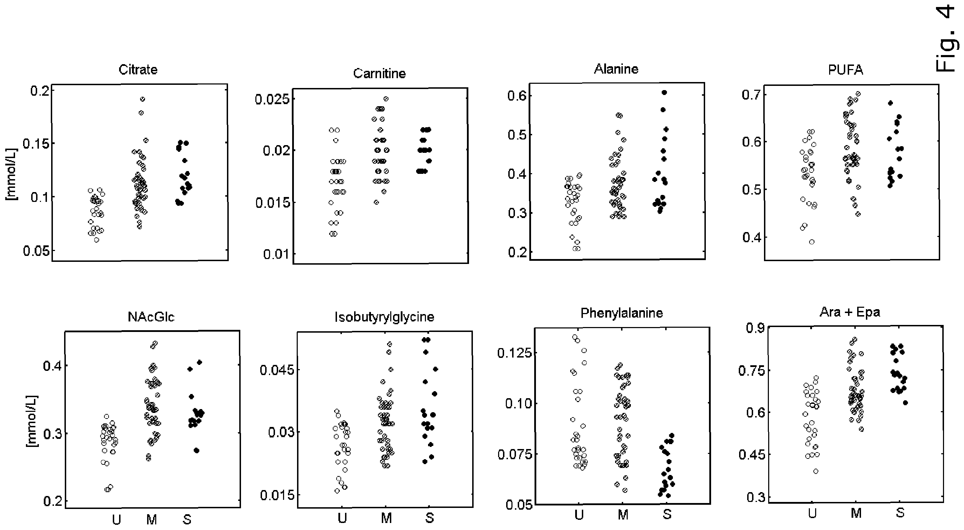

Figure 4 shows the distribution of metabolite concentrations in the groups of unaffected (U), mildly (M) and severely (S) affected patients by hypoxemia measured on the first morning after weaning from CPB.

*Figure 5*

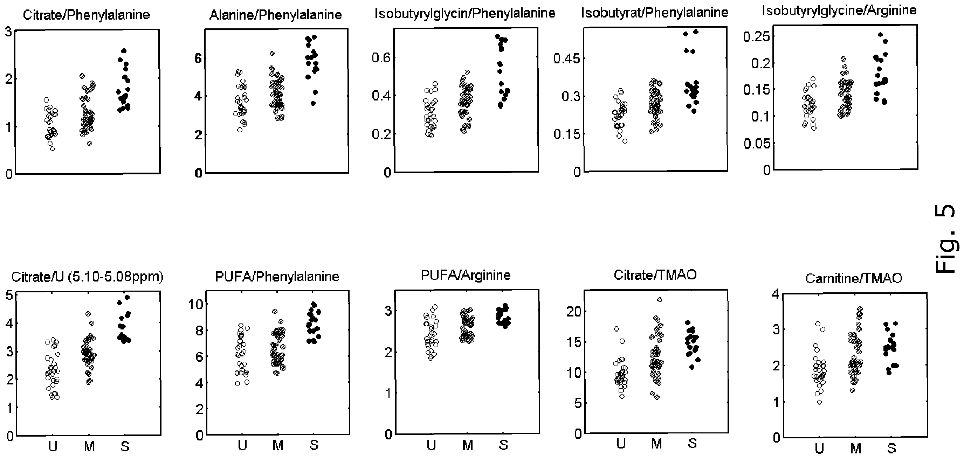

Figure 5 shows the distribution of the most significant metabolic ratios found differentiating the three patient groups (unaffected (U), mildly (M) and severely (S)).

*Figure 6*

Workflow of the computer-aided method described in the invention. **The left box** represents standard processing techniques applied for NMR and metabonomics studies that are imbedded in packages connected to the NMR system. **The right box** represents the present workflow and it contains a script/test that performs: 1) automatic preprocessing of spectral data (generalized log transformation, normalization, scaling), 2) feature elimination of spectral regions not correlating to $PaO_2$ values used in diagnosis three days postoperatively, 3) multivariate statistical data analysis with the use of the machine learning technique PLS regression to predict the oxygenation values ($PaO_2$ or $PaO_2/FiO_2$ in kPa), 4) calibration and validation of PLS regression results, 5) visualization of the results including the predicted details of a patient based on the models used as references, 6) multivariate statistical data analysis with the use of the machine learning technique PLS-DA to screen for disease, to asses in diagnosis (hypoxemia or none), and to determine the risk of ARDS, 7) calibration and validation of PLS-DA results, 8) visualization of the results including the predicted details of a patient based on the models used as reference. Because the script/program contains 500 spectra as references run on serum samples collected at different time points (after thoracotomy (skin incision) but before coupling to the CPB, just after weaning from CPB (0 hours), 2 hours, 4 hours and 8 hours post-CPB)), it can also be applicable to monitor the effect of different treatment and therapeutic interventions and to determine if a patient condition is in flare or remission after a treatment has been initiated.

*Figure 7*

Representative example of regions of interest found correlating to the degree of hypoxemia determined three days postoperatively by the standard clinical test. The numbered regions corresponds to 1) Histidine, 2) Phenylalanine, 3) PUFA, 4) Carnitine, 5) TMAO, 6) Citrate, 7) N-AcGlc, and 8) Alanine.

*Figure 8*

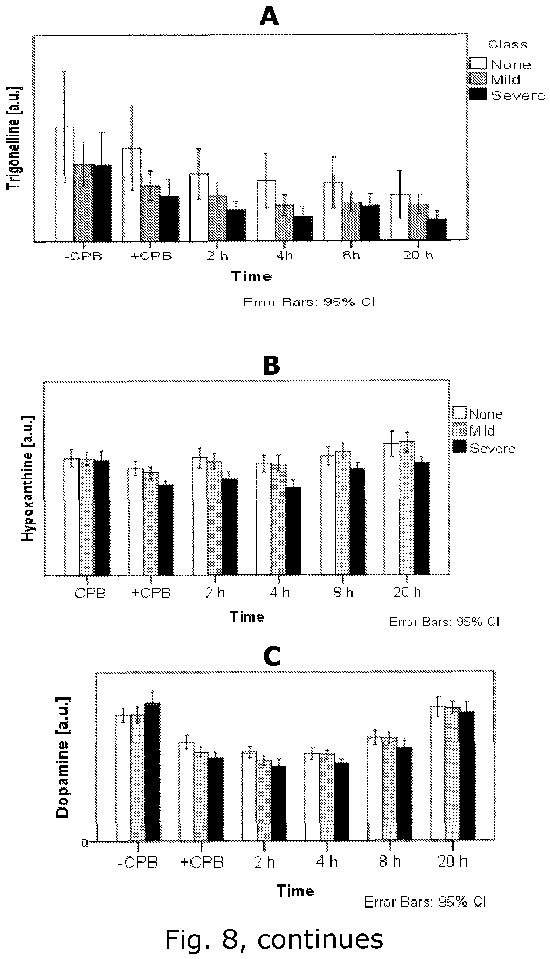

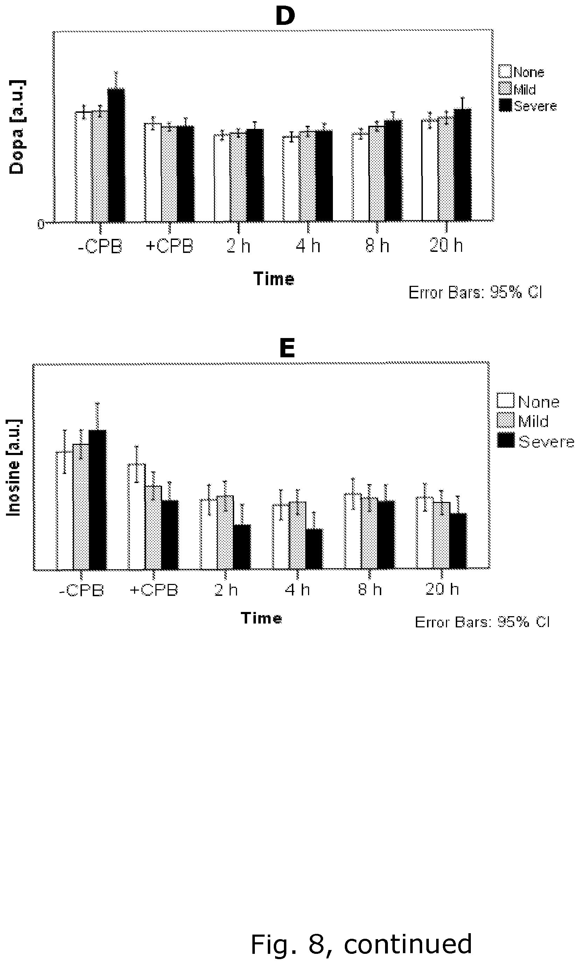

Figure 8 shows the relative concentration of metabolites represented as histogram plots with the mean and standard deviation indicated by the 95% confidence intervals.

A) Trigonelline; B) Hypoxanthine; C) Dopamine; D) Dihydroxyphenylalanine (Dopa); and E) Inosine. '-CPB' = after sternotomy but before CPB; '+CPB' = after weaning from CPB (0 hours); '2 h' = 2 hours after weaning from CPB; '4 h'= 4 hours after weaning from CPB; '8 h'= 8 hours after weaning from CPB; '20 h'= 20 hours after weaning from CPB. "None" = patients not presenting hypoxemia (unaffected; "Mild" = patients affected mildly by hypoxemia; and "Severe" = patients affected severely by hypoxemia.

The present invention will now be described in more detail in the following sections.

**Detailed description of the invention**

Definitions

Prior to discussing the present invention in detail, the following terms and conventions will first be defined:

*Hypoxemia*

Hypoxemia is a dangerous condition where oxygen content in arterial blood drops below normal levels. A decrease in oxygen delivery will affect lungs, brain, liver, and other organs, which in the most severe condition can lead to organ failure and even death. The most severe hypoxemia is seen in patients suffering acute respiratory distress syndrome (ARDS), which has a mortality rate of 20-40%. In the present context, hypoxemia is defined as the ratio between the partial pressure of arterial oxygen ($PaO_2$) and the fraction of inspired oxygen ($FiO_2$). Patients presenting $PaO_2/FiO_2$ values below 40 kPa (300 mmHg) are considered to have acute lung injury. The lower the value, the more severe the condition.

*Acute Lung Injury*

Acute lung injury (ALI) is a heterogeneous disease, which is defined by the acute onset of hypoxemic respiratory failure, with pulmonary infiltrates on chest x-ray due to non-cardiogenic pulmonary edema. The $PaO_2/FiO_2$ values in patients with ALI are below 40 kPa (300 mmHg); where $PaO_2$ is the partial pressure of oxygen in arterial blood, and $FiO_2$ is the fraction of inspired $O_2$.

*Cardiopulmonary bypass (CPB)*

Cardiopulmonary bypass (CPB) is a technique that temporarily takes over the function of the heart and lungs during cardiac surgery, maintaining the circulation of blood and the gas exchange. The CPB itself is often referred to as a heart-lung machine or "the pump".

*Reference level*

In the context of the present invention, the term "reference level" relates to a standard in relation to a quantity, which other values or characteristics can be compared to.

In one embodiment of the present invention, it is possible to determine a reference level by investigating the abundance of one or more of the biomarkers according to the invention in (blood) samples from subjects did not develop hypoxemia under the same surgical condition. By applying different statistical means, such as multivariate analysis, one or more reference levels can be calculated.

Based on these results a cut-off may be obtained that shows the relationship between the level(s) detected and patients at risk. The cut-off can thereby be used to determine the amount of the one or more biomarkers, which corresponds to for instance an increased risk of developing hypoxemia.

*Risk Assessment*

The present inventors have successfully developed a new method to predict the risk for developing hypoxemia for a patient several days in advance. The results presented in the examples show that the described biomarkers (alone or in combination) appear to be efficient markers for determining whether a patient has an increased risk of developing postoperative hypoxemia.

To determine whether a patient has an increased risk of developing hypoxemia due to related pulmonary complications such as acute lung injury, a cut-off must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each patient.

Statistics enables evaluation of the significance of each metabolite level. Commonly used statistical tests applied to a data set include t-test, f-test or even more advanced tests and methods of comparing data. Using such a test or method enables the determination of whether two or more samples are significantly different or not.

The significance may be determined by the standard statistical methodology known by the person skilled in the art.

The chosen reference level may be changed depending on the mammal/subject for which the test is applied.

Preferably the subject according to the invention is a human subject.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not sick.

Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

*Sensitivity*

$$Sensitivity = TP/ (TP + FN) = \text{number of true positive assessments/ number of all samples from patients with disease.}$$

As used herein the sensitivity refers to the measures of the proportion of actual positives which are correctly identified as such - in analogy with a diagnostic test, i.e. the percentage of people having $PaO_2$ below normal who are identified as having $PaO_2$ below normal.

*Specificity*

$$Specificity = TN/ (TN+ FP) = \text{number of true negative assessments/ number of all samples from controls.}$$

As used herein the specificity refers to measures of the proportion of negatives which are correctly identified - i.e. the percentage of mammals or people having $PaO_2$ at a normal level who are identified as having $PaO_2$ at a normal level. The relationship between both sensitivity and specificity can be assessed by the ROC curve. This graphical representation helps to decide the optimal model through determining the best threshold -or cut-off for a diagnostic test or a biomarker candidate.

As will be generally understood by those skilled in the art, methods for screening are processes of decision-making and therefore the chosen specificity and sensitivity depend on what is considered to be the optimal outcome by a given institution/clinical personnel.

It would be obvious for a person skilled in the art that it may be advantageous to select a higher sensitivity at the expense of lower specificity in most cases, to identify as many patients with disease as possible.

In a preferred embodiment, the invention relates to a method with a high specificity, such as at least 70%, such as at least 80%, such as at least 90%, such as at least 95%, such as 100%.

In another preferred embodiment, the invention relates to a method with a high sensitivity, such as at least 80%, such as at least 90%, such as 100%.

Another part of the invention relates to a method wherein the ratio between at least two markers is used to predict whether or not a subject is at risk of developing hypoxemia.

Method for determining the risk of developing hypoxemia

As described above, the present inventors have identified a novel set of biomarkers, which can be used to determine the risk of developing hypoxemia for a subject having had open cardiac surgery with the use of cardiopulmonary bypass (CPB). Thus, the first aspect of the invention relates to a method for determining the risk of developing hypoxemia, the method comprising

- providing a biological sample (said sample has been provided) from a subject, said subject having had open cardiac surgery with the use of cardiopulmonary bypass (CPB), wherein said sample has been provided from said subject between start of surgery (defined as skin incision) and 72 hours after detachment from the CPB circuit;

- determining the level of at least one biomarker selected from the group consisting of carnitine, citrate, isobutyrylglycine, N-Acetyl-Glucosamine, arachidonic acid, and isobutyric acid; and/or

- determining the ratio between levels of markers of at least one ratio selected from the group of ratios selected from citrate/phenylalanine, PUFA/phenylalanine, alanine/phenylalanine, citrate/Trimethylamine-N-oxide (TMAO), carnitine/TMAO, isobutyric acid/phenylalanine, and isobutyrylglycine/arginine;

- comparing said levels of one or more biomarkers or ratios to one or more reference levels;

- determining that said subject is at risk of developing hypoxemia if said level of one or more biomarkers are above the one or more reference levels, or determining that said subject is not at risk of developing hypoxemia, if said one or more levels are equal to or below the one or more reference levels; and/or

- determining that said subject is at risk of developing hypoxemia if said one or more ratios between levels of biomarkers are above the one or more reference levels, or determining that said subject is not at risk of developing hypoxemia, if said level of one or more ratios between levels of biomarkers are equal to or below the one or more reference levels.

A second aspect of the invention relates to a method for determining the risk of developing hypoxemia, the method comprising

- providing a biological sample, such as serum, plasma, or full blod (said sample has been provided) from a subject, said subject having had open cardiac surgery with the use of cardiopulmonary bypass (CPB), wherein said sample has been provided from said subject between the time of weaning from the CPB (0 hours) (or start of surgery (defined as time of skin incision)) and 20 hours after detachment/weaning from the CPB circuit;

- determining the level of at least one biomarker selected from the group consisting of Trigonelline; Hypoxanthine; Dopamine; Dihydroxyphenylalanine (Dopa); and Inosine;

- comparing said levels of one or more biomarkers to one or more reference (metabolic) levels;

- determining that said subject is at risk of developing hypoxemia if said level of at least one of the metabolites Trigonelline, Hypoxanthine, Dopamine, and Inosine are below the one or more reference levels, and/or determining that said subject is at risk of developing hypoxemia, if said level of Dihydroxyphenylalanine (Dopa) is above the one or more reference levels; or

- determining that said subject is not risk of developing hypoxemia if said level of at least one of the metabolites Trigonelline, Hypoxanthine, Dopamine, and Inosine are above or equal to the one or more reference levels, and/or determining that said subject is not at risk of developing hypoxemia, if said level of Dihydroxyphenylalanine (Dopa) is below or equal to the one or more reference levels.

For said second aspect, the levels of the biomarkes are preferably determined within 20 hours from detachment from the CPB circuit, more preferably within 4 hours, and even more preferably within few minutes after detachment/weaning from the CPB circuit, such as with in 10 minutes, such as within 5 minutes or such as within 2 minutes.

Because hypoxemia normally develops within 3-7 days following an initial insult such as CPB, an early prediction of the condition is desirable. To achieve an early prediction, it may be advantageous to obtain/provide (or have provided) the (blood) sample at several time points soon after surgery, within an interval of 2 days postoperatively. Thus, in an embodiment, said sample is provided (or has been provided) from said subject within 48 hours after detachment from the CPB circuit, such as within 30 hours, such as within 20 hours, such as within 16 hours, such as within 10 hours, such as within 8 hours, such as within 5 hours, such as within 4 hours, such as within 2 hours, such as within 1 hour, or just after /weaning from the CPB circuit. In another embodiment, said sample is provided (or has been provided) from said subject (e.g. 2 hours or 90 minutes, or 60 minutes) before detachment from the CPB circuit until 2 hours after detachment from the CPB circuit.

In example 2, samples were obtained first morning (16 hours) after weaning from the CPB. In example 5, samples were obtained at different time points after detachment from the CPB (t=0 until t=8 hours).

In example 6 it is shown that samples were obtained at different time points after sternotomy but before CPB and after weaning from the CPB (t=-CPB, t= +CPB (0 hours) until t=20 hours). All examples provide strong predictive values in relation to the risk of developing hypoxemia.

It is of course to be understood that the levels in the indicated ratio could also be mathematically reversed, resulting in the indicated risk be the opposite. This is considered to also form part of the present invention.

The predicted hypoxemia may be predicted to occur within different time periods. Thus, in another embodiment, hypoxemia is predicted to occur within a week from initiation of cardiac surgery, such as within 72 hours, such as within 60 hours, or such as within 48 hours, preferably within 72 hours.

In an embodiment said sample has been provided from said subject after sternotomy but before detachment from the CPB circuit until 2 hours after weaning from the CPB circuit

When the sample is obtained, the subject will normally have normal oxygen levels ($PaO_2/FiO_2$> 40 kPa (> 300 mmHg)) and thus not suffer from hypoxemia. Thus, in a further embodiment said subject has normal blood oxygenation levels at the time the sample was obtained, such as having a $PaO_2/FiO_2$ equal to or above 40 kPa (300 mmHg). To increase the predictive value of the method, preferably more than one biomarker is determined by the method of the invention. Thus, in an embodiment, the levels of at least two biomarkers are determined. In another embodiment, the levels of at least three biomarkers, such as at least four biomarkers, such as at least five biomarkers, such as at least six biomarkers are determined.

In another embodiment, at least the ratios between citrate/phenylalanine, PUFA/phenylalanine and alanine/phenylalanine are determined.

In another embodiment, at least the levels of carnitine and citrate are determined. In yet another embodiment at least the levels of carnitine and citrate, and the citrate/U (unidentified metabolite occurring at 5.10-5.08 ppm on our NMR spectrum) ratio are determined. In yet another embodiment, at least the levels of carnitine, citrate, isobutyrylglycine, N-acetyl-glucosamine, arachidonic acid, and isobutyric acid are determined.

The type of sample material may vary. Thus, in an embodiment, the sample is a blood sample, such as whole blood, serum and/or plasma. The blood sample may have been provided from different locations from the subject. In an embodiment, the blood sample has been obtained from the left atrium (LA), the pulmonary artery (PA), venous blood and/or arterial blood.

The determination of the presence and levels of these metabolites in a sample can be performed by many different methods. Thus, in an embodiment, said level of biomarkers are determined by a method selected from the group consisting of mass spectrometry (GC-MS, LC-MS), HPLC, Raman, NIR and NMR spectroscopy. The skilled person may identify other methods, which may be used.

The severity of hypoxemia may vary. In an embodiment, said subject is determined at risk of having mild hypoxemia. In the present context, patients with mild hypoxemia have a $PaO_2/FiO_2$ in the range between 40 -26.6 kPa (300-200 mmHg).

In another embodiment, said subject is determined to be at risk of having severe hypoxemia. In the present context, severe hypoxemia is defined as patients having a $PaO_2/FiO_2$ <26.6 kPa (200 mmHg).

The type of open cardiac surgery may vary. Thus, in an embodiment, said open heart surgery is selected from the group consisting of coronary artery bypass grafting and valve surgery.

The reference levels may be determined in different ways. In an embodiment, said one or more reference levels are determined from levels of said biomarkers in blood samples from subjects having had open heart surgery with the use of CPB, who did not develop hypoxemia postoperatively as diagnosed by standard diagnostic tests at least 3 days postoperatively.

The predictive value of the method may be further increased by including even further biomarkers in the assay. Table 4 lists some of the biomarkers identified as also having a predictive value, albeit with a lower predictive power. Thus, in an embodiment said method further comprises determining the level of at least one marker selected from the group listed in Table 4.

In that case determination according to the method of the invention would be:

- determining that said subject is at risk of developing hypoxemia if said one or more levels of biomarkers are below/above the one or more reference levels, or determining that said subject is not at risk of developing hypoxemia, if said one or more levels of biomarkers are equal to, above/below the one or more reference levels.

From table 4, the skilled person will be able to adapt the method accordingly.

Initiation of treatment based on prediction

If a subject is considered at risk of developing hypoxemia it may be advantageous to initiate a preventive treatment to avoid hypoxemia occurring. Thus, another embodiment of the invention, the method further comprises, for a subject considered at risk of early developing hypoxemia,

- providing to said subject an earlier treatment protocol for the prevention of hypoxemia, such as anti-oxidant treatment, anti-inflammatory treatment, glucose administration, insulin treatment, oxygen supplementation, respiratory support and/or fluid treatment;
and/or;

- having said subject under observation for the development of hypoxemia.

Method for evaluating the efficacy of treatment to prevent hypoxemia

By having identified biomarkers allowing early prediction of hypoxemia in a subject it also becomes possible to test/evaluate possible treatment protocols which may prevent the development of hypoxemia. Thus, another aspect of the invention relates to a method for evaluating the efficacy of treatment to prevent hypoxemia, the method comprising

- completing the method according to the first aspect of the invention; and
- for a subject considered at risk of developing hypoxemia, evaluating whether a treatment protocol for the prevention of hypoxemia, results in that said subject not developing hypoxemia within one week after weaning from the CPB circuit.

In embodiments, following Example 5 below, the method comprises applying NMR spectroscopy on said biological sample, e.g. 1D NMR, so as to arrive at a resulting spectrum covering a spectral range representative of said biomarkers, and applying a computer-based algorithm in response, so as to determine an output indicative of said subject being at risk of developing hypoxemia. Especially, said biological sample, such as a blood sample, has been provided from said subject between start of surgery (defined as skin incision) until 24 hours after weaning from the CPB circuit (preferably until 8 hours). Preferably the sample is a serum sample.

More specifically, said biological sample has been provided from said subject between a time during surgery, such as from weaning from the CPB circuit until 8 hours after weaning from the CPB circuit. In the present context, end of surgery is defined as time of skin closure (such as by stitching), which will often be 1-2 hours after weaning from the CPB. As shown in table 5 and 6, strong predictive values for the risk of developing hypoxemia are obtainable from just after weaning from the CPB circuit.

The method may comprise selecting from the resulting spectrum a plurality of spectral intervals (or regions), wherein a combination of spectral intervals are selected to cover at least spectral intervals indicative of at least one of: arginine, phenylalanine, PUFA, alanine, TMAO, carnitine, isobutyric acid, isobutyrylglycine and citrate. Especially, the combination of spectral intervals may be selected to cover all of: arginine, phenylalanine, PUFA, alanine, TMAO, carnitine, isobutyric acid, isobutyrylglycine and citrate. These compunds are part of the ratios considered the most relevant according to the present invention. In a further embodiment, the combination of spectral intervals are selected to cover at least spectral intervals indicative of at least one of: carnitine, isobutyrylglycine, N-Acetyl-Glucosamine, arachidonic and eicosapentanoic acid, isobutyric acid and citrate. In yet a further embodiment, the combination of spectral intervals may be selected to cover all of: carnitine, isobutyrylglycine, N-Acetyl-Glucosamine, arachidonic and eicosapentanoic acid, isobutyric acid, and citrate.

The combination of spectral intervals may be selected to cover at least spectral intervals indicative of at least 14 metabolites, such as 14-19 metabolites. The method may comprise performing a computer-based statistical analysis of said combination of spectral intervals, such as a computer-based statistical analysis involving applying a machine learning algorithm. The method may comprise presenting a visual output indicative of said subject being at risk of developing hypoxemia, e.g. a presentation of data allowing a medical person to determine if said subject is or is not at risk of developing hypoxemia.

Following Example 5, an aspect of the invention may be defined as a method for determining the risk of developing hypoxemia, the method comprising

- providing a biological sample from a subject, said subject having had open cardiac surgery with the use of cardiopulmonary bypass (CPB), wherein said sample has been provided from said subject between start of surgery (defined as skin incision) and 72 hours after weaning from the CPB circuit;
- applying NMR spectroscopy on said biological sample, such as performing a 1D CPMG NMR analysis, so as to arrive at a resulting spectrum representative of the level of at least one biomarker selected from the group consisting of carnitine, isobutyrylglycine, N-Acetyl-Glucosamine, arachidonic and eicosapentanoic acid, isobutyric acid and citrate;

and/or

- said resulting spectrum being representative of the ratio between levels of biomarkers of at least one ratio selected from the group of ratios selected from citrate/phenylalanine, PUFA/phenylalanine, alanine/phenylalanine, citrate/ TMAO, carnitine/TMAO, isobutyric acid/phenylalanine, and isobutyrylglycine/arginine;

  and

- applying a computer-based algorithm on said resulting spectrum involving a comparison with reference data, so as to determine if said subject is at risk of developing hypoxemia and/or so as to determine if said subject is not at risk of developing hypoxemia.

Further, following Example 5, an aspect of the invention may be defined as a computer program product having instructions which, when executed on a processor cause the processor to

- receive data representing a resulting spectrum from an NMR spectroscope representative of the level of at least one biomarker selected from the group consisting of carnitine, isobutyrylglycine, N-Acetyl-Glucosamine, arachidonic and eicosapentanoic acid, isobutyric acid and citrate;
  and/or

- said resulting spectrum being representative of the ratio between levels of biomarkers of at least one ratio selected from the group of ratios selected from citrate/phenylalanine, PUFA/phenylalanine, alanine/phenylalanine, citrate/ TMAO, carnitine/TMAO, isobutyric acid/phenylalanine, and isobutyrylglycine/arginine;

  and

- to apply a processing algorithm on said resulting spectrum involving a comparison with reference data, so as to determine if said subject is at risk of developing hypoxemia and/or so as to determine if said subject is not at risk of developing hypoxemia.

Such computer program product may be designed for execution on a portable computer, a server, partly or fully integrated with software dedicated for an NMR spectrometer etc. The resulting spectrum determined by the NMR spectrometer may be transferred in wire or wireless form to a separate processing device arranged to execute the computer program product. Especially, the computer program product may be present on a computer readable medium.

**Other aspects of the invention**

The method according to the invention is believed also to find use in other medical conditions being triggers of acute lung injury. These conditions are related to the present medical condition (open heart surgery with the use of CPB), by also relating to acute lung injury as described previously.

These conditions may be sepsis, pneumonia, severe trauma, inhalation of toxic agents, aspiration of gastric contents, major surgery, and transfusions.

Thus, in an additional aspect, the invention relates to a method for determining the risk of developing hypoxemia, the method comprising

- providing a biological sample from a subject considered at risk of developing hypoxemia;

- determining the level of at least one biomarker selected from the group consisting of carnitine, citrate, isobutyrylglycine, N-acetyl-glucosamine, arachidonic acid, and isobutyric acid;
  and/or

- determining the ratio between levels of markers of at least one ratio selected from the group of ratios selected from citrate/phenylalanine, PUFA/phenylalanine, alanine/phenylalanine, citrate/TMAO, carnitine/TMAO, isobutyric acid/phenylalanine, and isobutyrylglycine/arginine;

- comparing said levels of one or more biomarkers or ratios to one or more reference levels;

- determining that said subject is at risk of developing hypoxemia if said level of one or more biomarkers are above the one or more reference levels, or determining that said subject is not at risk of developing hypoxemia, if said one or more levels are equal to or below the one or more reference levels;
  and/or

- determining that said subject is at risk of developing hypoxemia if said one or more ratios between levels of biomarkers are above the one or more reference levels, or determining that said subject is not at risk of developing hypoxemia,

if said level of one or more ratios between levels of biomarkers are equal to or below the one or more reference levels.

In a specific embodiment, said subject experiences sepsis, pneumonia, severe trauma such as cardiac, aortic, thoracic, and spinal surgery, traumatic brain injury, lung transplantation, multiple traumas and transfusions, or has inhaled toxic agents, or aspired gastric contents.

It should be noted that embodiments and features described in the context of one of the aspects of the present invention also apply to the other aspects of the invention.

The invention will now be described in further detail in the following non-limiting examples.

## Examples

### Example 1

Methods employed for identifying biomarkers, which are early biomarkers for the development of hypoxemia.

*Patient group and clinical outcome*

Serum samples were obtained from forty-seven (n=47) patients undergoing coronary artery bypass grafting (CABG). Three days postoperatively, 15 patients showed no signs of hypoxemia; while 32 developed hypoxemia with $PaO_2$ below normal values.

*Samples preparation*

To avoid preanalytical bias due to sample collection, all blood samples were collected and prepared by the same person. Blood samples were obtained from both the left atrium (LA) and pulmonary artery (PA) precisely 16h after weaning from the CPB circuit. To obtain serum samples, blood was allowed to clot at room temperature for 30 minutes, and was subsequently centrifuged at 3000 rpm for 10 minutes. Aliquots of LA and PA serum were immediately stored at -80 °C until assayed.

*Sample preparation for NMR*

Prior to NMR measurements, samples were thawed for 2h at 4°C, vortexed, and centrifuged for 5 minutes at 4°C and 14000 rpm to remove cells and other precipitated materials. Aliquots of 400 $\mu$L of supernatant were mixed with 200 $\mu$L 0.2 M phosphate buffer (pH 7.4, uncorrected meter reading) in $^2H_2O$ (99% $^2H$) to minimize variations in pH and to reduce serum viscosity. Throughout the whole process, the samples were kept on ice. The mixture was pipetted into a 5-mm NMR tube and NMR analysis was performed.

*NMR Measurements*

Spectra were acquired on a Bruker DRX-600 NMR spectrometer (Bruker BioSpin, Germany and Switzerland) equipped with a TXI (hydrogen, carbon, nitrogen) probe (Bruker BioSpin, Switzerland) operating at 600.13 MHz for $^1H$. The experiments were acquired at a constant temperature of 310.1 K (37 °C). For the analysis, a $T_2$ relaxation-edited Carr-Purcell-Meiboom-Gill (CPMG) experiment was used. This experiment attenuates broad signals from slowly tumbling proteins and lipoproteins. 128 free induction decays (FIDs) were collected with 32768 complex data points over a spectral width of 11.97 ppm and an acquisition time of 2.28s. A relaxation delay of 2s was applied between each FID during which weak continuous wave irradiation ($\gamma Bi/2\pi$ = 26.6 Hz) was applied at the water frequency (presaturation). The total spin-echo relaxation delay was 67.4 ms and consisted of ($\tau$-$\pi$-$\tau$) elements, where $\tau$ is a delay of 0.4ms and $\pi$ is a 180° pulse of 22$\mu$s length. Spectral processing was carried out in TopSpin version 2.1 (Bruker BioSpin, Germany). Prior to Fourier transformation, exponential multiplication corresponding to a line broadening of 0.3 Hz was applied to the FIDs, which were further zero-filled by a factor of 2 to double the number of points. Spectra were manually phase- and baseline-corrected, and the methyl signal of lactate was used as chemical shift reference at 1.33 ppm.

For metabolite identification, three types of two-dimensional (2D) experiments were acquired. 2D J-resolved $^1H$-NMR experiments, with water pre-saturation during a 2s relaxation delay were acquired with 8 FIDs for each of the 80 increments. Spectral width was set to 11.6 ppm and 54.7 Hz in F2 and F1 direction, respectively. Prior to 2D-Fourier transformation, both dimensions were multiplied by an unshifted sine bell function and the number of points was doubled by zero-filling. Further, spectra were tilted by 45° and symmetrized along the F1 axis. After processing, the skyline projections of 2D J-resolved spectra were manually baseline corrected and calibrated using the center of the methyl signal of lactate. 2D homonuclear $^1H$,$^1H$-TOCSY (Total Correlation Spectroscopy) and $^1H$,$^{13}C$-HSQC (Heteronuclear

Single Quantum Coherence) spectra with presaturation were run on representative samples, with different number of FIDs, increments, spectral widths and mixing times in order to focus on different spectral regions. Information obtained from these spectra was used to find matching metabolites in the Human Metabolome Database. Further metabolite assignments were performed using AMIX (v. 3.9.10, Bruker BioSpin), BRUKER bbiorefcode databases (2.7.0-2.7.3), and literature.

*Quantification of NMR data*

This was performed using the AMIX multi integration tool. Peaks of interest were integrated using the line shape analysis option with a fixed noise factor of 3.5 and a line shape threshold of 0.01. Peaks were integrated using the sum of all points in the region as the integration mode, and normalized to the glucose metabolite region (3.52 ppm (H-5 of $\beta$-glucose) and 5.24 ppm (H-1 of $\alpha$-glucose)). A 36:64 equilibrium distribution between $\alpha$- and $\beta$-glucose was used for the calculations. To calculate the concentration of a given metabolite, we used the following formula:

$$C_X [mM] = \frac{I_X}{N_X} \cdot C_{Glucose} \cdot 0.64 \frac{N_{\beta-Glucose}}{I_{\beta-Glucose}}$$

where $C_X$ is metabolite concentration in mM, $I_X$ is the integral of the metabolite $^1$H peak, $N_X$ is the number of protons contributing to the metabolite $^1$H peak, $C_{Glucose}$ is the chemically determined glucose concentration, $0.64N_{\beta-Glucose}$ is the number of protons contributing to the $\beta$-glucose signal (at 3.52 ppm) used as reference multiplied by 0.64 (the mole fraction of $\beta$-glucose), and $I_{\beta-Glucose}$ is the integral of the $\beta$-glucose signal at 3.52 ppm.

*NMR data preprocessing*

CPMG spectra were converted into an n-by-m matrix (n= 94 LA and PA samples from 47 patients, m= 8700 equal buckets of 0.001 ppm width) in AMIX (Analysis of MIXtures software package, version 3.9.10, Bruker BioSpin, Germany) using the region between $\delta$ 9 to 0 ppm, and excluding the water signal region between 4.80-4.5 ppm. Further processing and multivariate modelling were carried out in MATLAB R2011b[56] coupled with PLS-Toolbox 6.5 (Eigenvector Research, Wenatchee, WA). Binned data was *i*Coshifted[57], normalized to lactate concentration measured 16h after surgery, log transformed, and autoscaled.

*Multivariate modelling*

Several mathematical and statistical modelling approaches were employed to relate the pre-processed NMR data to disease phenotypes. Partial least-squares discriminant analysis (PLS-DA) was applied to evaluate the diagnostic possibilities of NMR and to discover biomarkers. The most important variables obtained by the method were identified and quantified.

*Statistics*

The Statistical Package and Services Solutions (SPSS) software v22.0 was used for the statistical analysis. Descriptive statistics were computed for each group and the quantitative data was summarized using mean, median, and standard deviation.
The Shapiro-Wilk normality test was applied followed by either parametric or nonparametric methods. When comparing metabolite differences between unaffected and hypoxemic patients ($PaO_2$< 8.4kPa, PaO2/FiO2 < 40 kPa (<300 mmHg)), either an independent t-test or Mann-Whitney U test was used. When comparing differences between the three groups (unaffected, mild, severe), either one way analysis of variance (ANOVA) with Tukey HSD or Dunnett T3 (depending on homogeneity of variance) multiple comparison post-hoc tests, or the Kruskal-Wallis test with Dunn's post-hoc test were applied. When comparing data between the left atrium and pulmonary artery, a paired t-test or the nonparametric analogue Wilcoxon-signed rank sum test was used. Significance was set at p<0.05.
In order to visualize global changes between groups, the means or medians (depending on data distribution) of each metabolite in the corresponding group were calculated. As such, metabolite fold changes were calculated as the ratio between mild and unaffected and severe and unaffected patients with the formula:

$$FC_{\frac{M \, (or \, S)}{U}} [\%] = \frac{M(or \, S) - U}{U} \cdot 100$$

Where FC = fold change in percentage, M= mild, U= unaffected, S= severe. Because the ratio between two metabolite concentrations may carry more information than the two metabolites alone, the freely available web-based tool ROCCET (ROC Curve Explorer & Tester)[27] was used to compute all possible pairs of metabolite combinations that could be related to later outcomes. Metabolite ratios exhibiting statistical significance (p<0.05) were further used as biomarker candidates.

The association between metabolites and $PaO_2$ values obtained 3 days postoperatively were assessed by Pearson's correlation analysis. ROC curves were established to determine the prognostic value of each biomarker in future diagnosis. The area under the curve (AUC) and corresponding 95% confidence intervals (CIs) are provided.

## Example 2

Metabolome screening reveals early signs of disease:

The systemic and pulmonary phenotypes were monitored by $^1$H Nuclear Magnetic Resonance (NMR) spectroscopy. A typical one-dimensional (1D) serum NMR spectrum is characterized by broad resonances from lipids and glycoproteins, and narrow resonances from glucose, lactate, and citrate, among others. Spectra of two samples collected on the first postoperative day (exactly 16h after weaning from CPB), one from a patient showing no signs of hypoxemia ($PaO_2$=10.7 kPa or 80.2 mmHg), and one from a patient developing hypoxemia ($PaO_2$=4.9 kPa or 36.7 mmHg), reveal differences in several signals, of which lipids are the most significant (Figure 1).

Since the metabolome mirrors environmental changes, we hypothesized that the disease could be reflected at the metabolic levels on the first day postoperatively. Therefore, we screened for possible associations between the metabolome and the hypoxemic scores ($PaO_2$) used in diagnosis. We have divided the 32 patients diagnosed with acute lung injury into patients developing mild and severe hypoxemia, to provide better understanding of underlying mechanisms. Subsequently, the differences between the three groups (unaffected, mild, and severe hypoxemia) were assessed by partial least-squares discriminant analysis (PLS-DA). The models displayed >95% accuracy, indicating the remarkable performance of our screening test.

## Example 3

The metabolic *fingerprints* found in the screening test were investigated further. 64 different metabolites were analysed, of which one could not be identified (U (5.10-5.08 ppm)). Perturbations in the levels of metabolites involved in normal cellular functioning (amino acids, carbohydrates, ketones), cellular signalling (1,2-diacylglycerol), inflammation (arachidonic and eicosapentanoic acid), cell membrane and alveolar surfactant components (fatty acids, cholesterols, phospholipids) were found crucial in the development of injury. Carnitine, arachidonic and eicosapentanoic acid, glycoprotein, citrate, and phenylalanine, among others, showed the highest fold changes, indicating their key roles in later outcomes. Most metabolites showed consistent trends from none-to-mild-to-severe acute lung injury (Figure 4), indicating their correlation to the degree of later pulmonary dysfunction and their possible function as predictive biomarkers.

The list of the most relevant biomarkers is listed in the tables below.

*Table 4: Most significant metabolites found correlating to the Partial pressure of $O_2$ ($PaO_2$) measured on the day of diagnosis (72h after weaning from CPB).*

| Pearson Correlation (Metabolites vs. $PaO_2$ (72h)) | | | | |
|---|---|---|---|---|
| | | Metabolites | Correlation | Sig. (2-tailed) |
| | 1 | Citrate/U(5.10-5.08 ppm) | -0.645** | 1.77E-06 |
| | 2 | Isobutyrylglycine/Phenylalanine | -0.609** | 8.86E-06 |
| | 3 | Isobutyrylglycine/Arginine | -0.602** | 1.20E-05 |
| | 4 | Citrate/Phenylalanine | -0.572** | 4.02E-05 |
| | 5 | PUFA/Phenylalanine | -0.562** | 5.83E-05 |
| | 6 | Alanine/Phenylalanine | -0.556** | 7.37E-05 |
| | 7 | Isobutyrate/Phenylalanine | -0.546** | 1.03E-04 |
| | 8 | PUFA/Arginine | -0.546** | 1.06E-04 |
| | 9 | Arachidonic (Ara) and Eicosapentanoic acid (Epa) | -0.509** | 3.62E-04 |

(continued)

| Pearson Correlation (Metabolites vs. PaO$_2$ (72h)) | | | |
|---|---|---|---|
| | Metabolites | Correlation | Sig. (2-tailed) |
| 10 | Isobutyrylglycine | -0.455** | 1.70E-03 |
| 11 | Citrate/TMAO | -0.452** | 1.85E-03 |
| 12 | Carnitine | -0.421** | 4.00E-03 |
| 13 | Citrate | -0.388** | 8.50E-03 |
| 14 | Carnitine/TMAO | -0.383** | 9.37E-03 |
| 15 | NAc-Glc | -0.330* | 2.69E-02 |
| 16 | Phenylalanine | 0.317* | 3.38E-02 |
| 17 | Alanine | -0.293 | 5.10E-02 |

Abbreviations: Sig.= significance level; U= unknown metabolite, TMAO: trimethylamine-N-oxide, 1,2-DAG= 1,2-diacylglucerol, GPE= glycerophosphoethanolamine, GPC= glycerophosphocholine, 3-HBA= 3-hydoxybutyric acid, VLDL= very low density lipoproteins, LDL= low density lipoproteins, HDL= high density lipoproteins, DAGPL= diacylglycerolphospholipid, UFA= unsaturated fatty acids, Free FA= free fatty acids, NAcGal= N-acetyl-galactose amine, NAc-Glc= N-acetyl-glucosamine, Lyso-PC= lysophosphatidylcholine. In the present context "PUFA" refers to polyunsaturated fatty acids ("PUFAs"). PUFAs are categorized according to the number and position of double bonds in the fatty acids according to an accepted nomenclature that is well known to those of ordinary skill in the art. Polyunsaturated omega-3/6 fatty acids (with ≥3 double bonds); e.g. eicosopentaenoic acid[EPA, 20:5 (n-3)], docosahexaenoic acid [DHA, 22:6(n-3)], linoleic (18:2 n-6), gamma-linolenic acid (18:3 n-6), alpha-linolenic (18:3 n-3) and stearidonic (18:4 n-3) acid.

*Table 2A: Most significant metabolites found predicting later outcomes by applying t-test; Hypoxemia vs. Unaffected Patients.*

| | Metabolites | Hypoxemia vs. Unaffected Patients | | |
|---|---|---|---|---|
| | | AUC | 95% C.I. AUC | Sig. |
| 1 | Carnitine | 0.90 | 0.79-0.979 | 6.96E-07 |
| 2 | Citrate/TMAO | 0.89 | 0.749-0.993 | 3.59E-06 |
| 3 | Citrate | 0.88 | 0.764-0.961 | 2.43E-05 |
| 4 | Carnitine/TMAO | 0.86 | 0.714-0.971 | 5.21E-05 |
| 5 | NAcGlc | 0.86 | 0.748-0.951 | 2.14E-04 |
| 6 | Citrate/Phenylalanine | 0.85 | 0.722-0.942 | 1.31E-04 |
| 7 | Citrate/ U (5.10-5.08 ppm) | 0.85 | 0.705-0.948 | 2.60E-05 |
| 8 | Isobutyrate/Phenylalanine | 0.84 | 0.676-0.935 | 5.64E-04 |
| 9 | Arachidonic and Eicosapentanoic acid | 0.82 | 0.688-0.953 | 5.95E-05 |
| 10 | Isobutyric acid | 0.79 | 0.63-0.899 | 2.31E-03 |
| 11 | Alanine/Phenylalanine | 0.78 | 0.633-0.908 | 2.94E-03 |
| 12 | Isobutyrylglycine/Arginine | 0.78 | 0.635-0.911 | 1.32E-03 |
| 13 | Isobutyrylglycine/Phenylalanine | 0.77 | 0.622-0.917 | 2.52E-03 |
| 14 | PUFA/Phenylalanine | 0.76 | 0.601-0.882 | 4.83E-03 |
| 15 | PUFA/Arginine | 0.76 | 0.594-0.894 | 2.87E-03 |
| 16 | Isobutyrylglycine | 0.75 | 0.603-0.879 | 3.65E-03 |

*Table 2B: Metabolites found significant in predicting later outcomes by applying t-test; Severe vs. Unaffected Patients.*

| Metabolites | Severe vs. Unaffected Patients | | |
|---|---|---|---|
| | AUC | 95% C.I. AUC | Sig. |
| Citrate/Phenylalanine | 1.00 | 1.00-1.00 | 1.06E-06 |
| Citrate/ U (5.10-5.08 ppm) | 1.00 | 1.00-1.00 | 2.71E-05 |
| Isobutyrate/Phenylalanine | 0.98 | 0.912-1.00 | 5.88E-05 |
| PUFA/Phenylalanine | 0.98 | 0.905-1.00 | 2.14E-05 |
| PUFA/Arginine | 0.97 | 0.881-1.00 | 3.85E-05 |
| Alanine/Phenylalanine | 0.97 | 0.865-1.00 | 1.45E-05 |
| Isobutyrylglycine/Arginine | 0.95 | 0.857-1.00 | 7.73E-05 |
| Isobutyrylglycine/Phenylalanine | 0.94 | 0.833-1.00 | 5.44E-05 |
| Arachidonic and Eicosapentanoic acid | 0.93 | 0.802-1.00 | 5.46E-04 |
| Citrate | 0.93 | 0.782-1.00 | 6.45E-04 |
| Carnitine | 0.90 | 0.762-1.00 | 8.75E-04 |
| Citrate/TMAO | 0.92 | 0.77-1.00 | 8.20E-05 |
| Carnitine/TMAO | 0.88 | 0.714-1.00 | 1.59E-03 |
| NAcGlc | 0.88 | 0.714-1.00 | 1.70E-02 |
| Abbreviations: AUC = area under the curve, C.I. = confidence level, U= unknown metabolite, TMAO = trimethylamine-N-oxide, NAcGlc = N-acethyl-glucosamine | | | |

<u>Conclusion</u>

A set of different biomarkers having predictive values for determining the risk of developing hypoxemia in patients undergoing open heart surgery with the use of CPB have been identified.

**Example 4**

<u>Early markers predict risk for hypoxemia</u>

Several serum metabolites measured 16h postoperatively were found to significantly correlate to $PaO_2$ measured 72h after weaning from CPB (Figure 4/Tables 1-2). Also, several metabolites showed strong predictive power for mild and severe hypoxemia with high area under the curves (AUC>0.8, p<0.0001) (tables 2A and 2B). This is the first study presenting predictive biomarkers of postoperative acute lung injury 56h before hypoxemia was diagnosed.

*Table 3: Metabolites considered most relevant (single marker or ratios).*

| Metabolites | Regulation considered indication of the risk of developing hypoxemia |
|---|---|
| Carnitine | Higher levels in hypoxemia |
| Citrate/TMAO | Higher ratio in hypoxemia |
| Citrate | Higher levels in hypoxemia |
| Carnitine/TMAO | Higher ratio in hypoxemia |
| O-NAc-Glc | Higher levels in hypoxemia |
| Citrate/ Phenylalanine | Higher ratio in hypoxemia |
| Isobutyric acid/ Phenylalanine | Higher ratio in hypoxemia |

(continued)

| Metabolites | Regulation considered indication of the risk of developing hypoxemia |
|---|---|
| Arachidonic acid (Ara, 20:4 $\omega$-6) and Eicosapentanoic acid (Epa, 20:5 $\omega$-3) | Higher levels in hypoxemia |
| Isobutyric acid | Higher levels in hypoxemia |
| Alanine/ Phenylalanine | Higher ratio in hypoxemia |
| Isobutyrylglycine/ Arginine | Higher ratio in hypoxemia |
| Isobutyrylglycine/ Phenylalanine | Higher ratio in hypoxemia |
| PUFA/ Phenylalanine | Higher ratio in hypoxemia |
| PUFA/Arginine | Higher ratio in hypoxemia |
| Isobutyrylglycine | Higher levels in hypoxemia |

Thus, the predictive markers presented in Table 3 were considered the most relevant metabolites identified. It is indicated in the table that higher levels are considered an indication of the risk of developing hypoxemia for the indicated biomarkers. Similarly, higher ratios between the indicated biomarkers are considered an indication of the risk of developing hypoxemia for the indicated biomarker ratios.

Table 4 contains a list of biomarkers also determined as being significantly different in patients at risk of developing hypoxemia, though with lower predictive values. One or more of these biomarkers may also be included in a method according to the invention to further increase the predictive value. The skilled person can adapt the method accordingly in relation to reference values.

*Table 4: Metabolites considered relevant (single marker).*

| Metabolites | |
|---|---|
| Phosphoglyceric acid (PGA) | Lower levels in hypoxemia |
| N-acetyl-galactosamine (NAcGal) | Higher levels in hypoxemia |
| Glycine | Higher levels in hypoxemia |
| Trimethylamine-N-oxide (TMAO) | Lower levels in hypoxemia |
| Dimethyl amine (DMA) | Higher levels in hypoxemia |
| Pyruvate | Higher levels in hypoxemia |
| Alanine | Higher levels in hypoxemia |
| Acetate | Higher levels in hypoxemia |
| Lysine | Higher levels in hypoxemia |
| Leucine, Isoleucine, Valine | Higher levels in hypoxemia |
| Malonic acid | Higher levels in hypoxemia |
| 3-Hydroxybutyric acid (3-HBA) | Higher levels in hypoxemia |
| Acetoacetate | Higher levels in hypoxemia |
| Formate | Higher levels in hypoxemia |
| Ethanol | Higher levels in hypoxemia |
| Isobutyrate | Higher levels in hypoxemia |
| 2-Ketobutyrate | Higher levels in hypoxemia |
| Carnitine | Higher levels in hypoxemia |
| Unsaturated fatty acids (UFA) | Higher levels in hypoxemia |

(continued)

| Metabolites | |
|---|---|
| Free fatty acids (FFA) + Adipate | Higher levels in hypoxemia |
| Arachidonic acid (Ara, 20:4 ω-6) and Eicosapentanoic acid (Epa, 20:5 ω-3) | Higher levels in hypoxemia |
| PUFA (Lipoic acid and related metabolites) | Higher levels in hypoxemia |
| Free Cholesterol | Higher levels in hypoxemia |
| Est. Cholesterol | Higher levels in hypoxemia |
| Lipoproteins (VLDL, LDL,HDL) | Higher levels in hypoxemia |
| Glycerophosphocholine (GPC) | Higher levels in hypoxemia |
| Lysophosphocholine (Lyso-PC) | Higher levels in hypoxemia |
| Choline | Higher levels in hypoxemia |
| Heparin | Lower levels in hypoxemia |
| Sphingomyelin | Higher levels in hypoxemia |
| Glycerol | Lower levels in hypoxemia |
| 1,2-Diacylglycerol (DAG) | Higher levels in hypoxemia |
| Diacylglycerophospholipid (DAGPL) | Higher levels in hypoxemia |
| Triacylglycerol (TAG) | Higher levels in hypoxemia |
| Plasmenyl | Higher levels in hypoxemia |
| Phosphoethanolamine (PE) | Higher levels in hypoxemia |
| Glycerophosphoethanolamine (GPE) | Higher levels in hypoxemia |
| Glutamate | Higher levels in hypoxemia |
| Glutamine | Lower levels in hypoxemia |
| Proline | Higher levels in hypoxemia |
| Creatine | Lower levels in hypoxemia |
| Phenylalanine | Lower levels in hypoxemia |
| Tyrosine | Lower levels in hypoxemia |
| Dopa | Lower levels in hypoxemia |
| Dopamine | Lower levels in hypoxemia |
| Histidine | Lower levels in hypoxemia |
| 1-Metylhistidine | Higher levels in hypoxemia |
| 3-Metylhistidine | Lower levels in hypoxemia |
| Urocanate | Lower levels in hypoxemia |
| Plasmalogen | Lower levels in hypoxemia |
| Xanthosine | Lower levels in hypoxemia |
| Hypoxanthine | Lower levels in hypoxemia |
| Arginine | Lower levels in hypoxemia |
| Citrulline | Lower levels in hypoxemia |
| U(5.10-5.08ppm) | Lower levels in hypoxemia |
| Phosphoenolpyruvate (PEP) | Higher levels in hypoxemia |

In an embodiment of the invention, the methods of the invention further includes determining the level of one or more of the biomarkers listed in Table 4. As can be seen in the right column, for some of the markers lower levels are indicative of the development of hypoxemia. Thus, for these specific markers the method will instead include

- determining that said subject is at risk of developing hypoxemia if said level of one or more biomarkers are below the one or more reference levels, or determining that said subject is not at risk of developing hypoxemia, if said one or more levels are equal to or above the one or more reference levels.

An example of such a biomarker is Phosphoglyceric acid (PGA) listed in table 4 above.

**Example 5**

Early indication of the risk of developing hypoxemia using NMR

**Patient cohort and diagnosis**

Patients scheduled for elective coronary artery bypass surgery (CABG) with use of cardiopulmonary bypass (CPB) were consecutively included in the study after informed consent was obtained (n=50).
The clinical diagnosis was based on the ratio between partial pressure of oxygen and fraction of inspired oxygen ($PaO_2/FiO_2$) calculated from $PaO_2$ measured in arterial blood samples collected from the radial artery 72 hours after weaning from CPB and in order to standardize the measurements, arterial blood samples were taken while patients were breathing atmospheric air for at least 10 minutes.

*Sample collection*

Blood was collected after the thorax was open but before patients were attached to the CPB circuit, just after CPB was removed (0 hours), 2 hours, 4 hours, and 8 hours after weaning from CPB.

*Sample Preparation*

Serum samples were thaw for 30 min. at 4°C, vortexed and subsequently centrifuged for 5 min at 12.100 g and 4°C. A total of 400 $\mu$L of the clear supernatant was mixed with 200 $\mu$L 0.2M phosphate buffer (pH 7.4 uncorrected meter reading, 99% $^2H_2O$) in a 5 mm NMR tube.

*NMR experiment*

Following, NMR spectra of the samples were recorded on a Avance-III 600 MHz NMR spectrometer (Bruker-BioSpin, Rheinstetten, Germany) equipped with a cryogenically cooled, triple-resonance, TCI probe, using a sample temperature of 298.1 K (25°C). Spectra acquisition was controlled using TopSpin 3.1 software (Bruker Biospin). A $T_2$ filter using the Carr-Purcell-Meiboom-Gill (CPMG)[21] pulse sequence with water presaturation was applied for each measurement. Each CPMG spectrum was acquired as 64k data points over a spectral width of 20 ppm, 256 scans, a fixed receiver gain (RG) of 203, and a relaxation delay (d1) of 4s.

*Spectra processing*

Spectral processing was carried out in TopSpin 3.1. Data was exponentially multiplied corresponding to a line broadening of 0.3Hz (CPMG), Fourier transformed, phase- and baseline corrected, and calibrated to the chemical shift of the methyl signal of L-alanine at 1.48 ppm. Spectra were reduced to regions of equal buckets (0.001 ppm) and the water region between $\delta$ 4.65 to $\delta$ 4.95 ppm was excluded in AMIX (Analysis of MIXtures software, v.3.9.10, Bruker BioSpin, Germany).

*The script*

Subsequently, data was exported in Matlab R2011b (The MathWorks, Inc., MA) programming environment. A script based on complex pattern recognition was written containing (1) spectral post-processing (generalized log trans-formation (gLog)[22] to enhance small signals in the spectrum, normalization, scaling and centering), (2) feature extraction by selecting the significant spectral regions correlating to later outcomes, (3) multivariate statistical analysis based on the machine learning algorithms PLS regression and PLS-DA, (4) validation, and (5) visualization of results.

For the feature extraction procedure, selecting the regions of interest (ROI) was achieved by dividing the data into intervals of 50 buckets and correlating each interval to the $PaO_2$ measured on the diagnostic day. The correlation was performed by the multivariate PLS regression analysis. Intervals with low correlation were discarded while significant intervals were further used in modeling. Extracting these distinctive features from serum spectra at each time point has shown to be the key characteristic of the success of our computer-aided diagnostic test. From a total of 10500 buckets, 7000-9500 buckets (depending on the time point analyzed) were removed as they did not show any correlation with the diagnostic $PaO_2$ values. Even the number of remaining features/buckets was higher than the number of samples (1000-3500 buckets vs. 100 samples per model); we considered that the data was not over-fitted, as these variables correspond to the information imbedded in 14-19 metabolites. These regions were consistently picked at each of the measured time points with minor variations. Taking into account that for each model we had 90-100 samples and the information from 14-19 metabolites, the models are considered not to be over-fitted. To assess the sensitivity and specificity of these selected regions, multivariate PLS-DA modelling was performed.

For the validation purpose the Venetian-Blinds cross-validation with 10 segmental splits was applied. Here 10 consecutive samples (5 patients in duplicate) were removed and a model was created. Following, the 10 samples were predicted. This procedure was repeated until all samples were removed once. To avoid overoptimistic interpretation and misleading results, we only refer to the cross-validated (CV) results of our modelling, however, the estimated (or calibrated) results are also provided. This validation procedure gave us an indicator of how well the model might work in predicting future samples.

A second level of model validation was performed by using permutation testing. Here, the models were tested for randomness, to show that no other model performed equally well or better than the main prediction model. After scrambling the $PaO_2$ values measured three days postoperatively (for PLS regression modeling) and the group labels (hypoxemia vs. unaffected; for the PLSDA modeling) 500 times and performing multivariate modelling, we compared the 'true' optimal PLS and PLSDA models with the permuted models. The "true" model performance were then statistically compared to the distribution of the permuted models, and a p-value was calculated by means of Wilcoxon's test. A p-value <0.001 was considered significant, meaning that the predictive power of our approach was significantly associated with $PaO_2$ and the diagnostic outcomes, and was not a false-positive association resulting from random prediction.

*Results*

In an embodiment of the invention the method is meant to be applied as follow; when a patient undergoes cardiac surgery, its blood sample is collected at one or several time points, and serum is extracted. Serum is mixed with phosphate buffer (see below for "Sample preparation"), and the sample is then run on NMR (see below for "NMR experiment"). Following this, the achieved spectrum is preprocessed (see below for "Spectra processing"), and the data has to be scanned through the script/test provided. As such, an embodiment includes the following steps:

1. Obtaining the blood sample
2. Preparing the sample
3. Running a 1D CPMG NMR
4. Applying standardized spectral processing to reduce artifacts (e.g. water suppression, pH shifts, baseline noise) (Figure 1, black box)
5. Fragmenting/binning the spectra to further minimize peak shifts
6. Exporting the data into the Matlab environment
7. Running the computed-aided diagnostic script/test (Figure 1, grey box)
8. Reading or interpreting the diagnosis and/or the effect of treatment

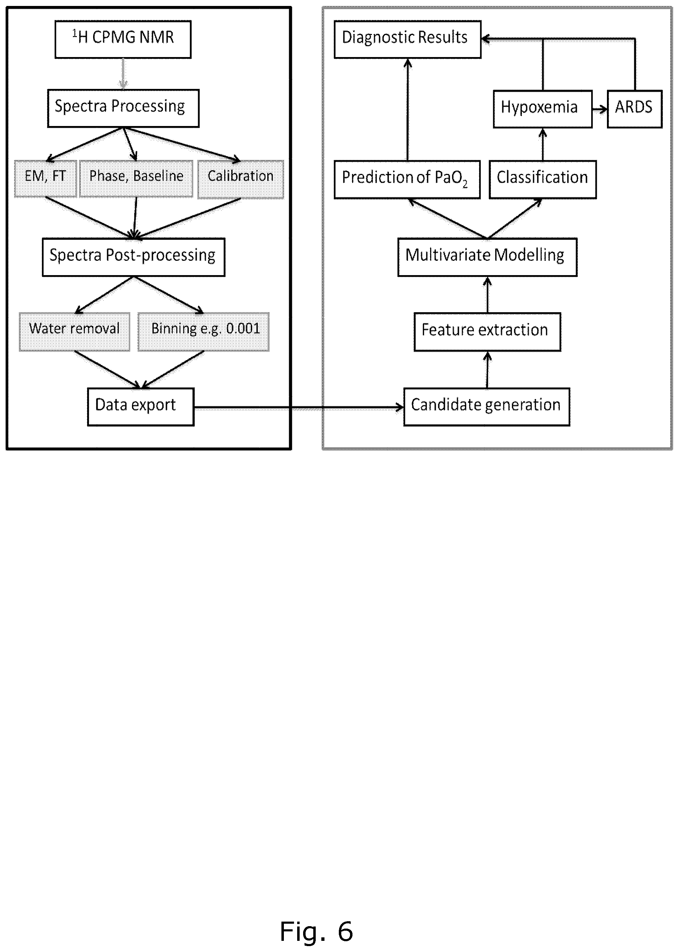

The script/test (Figure 6, gray box) is a computer-based diagnostic and/or monitoring algorithm based on machine learning. The script/test is optimized for 5 different time points, each time point containing 100 spectra run on serum samples collected before coupling to CPB, just after weaning from CPB but still during surgery, and 2, 4, and 8 hours postoperatively. First, the script post-processes the data (transformation, normalization, scaling, centering), then it selects the key ROI to be used for further diagnosis, it applies multivariate modelling (PLS or PLSDA) depending on the diagnostic interest ($PaO_2$ or hypoxemia vs. none), and it returns a certain score (for PLS) or a threshold level (for PLSDA) for each sample in the analysis. Finally, based on these scores or thresholds, a regression and a scores plot are provided and each sample's position is highlighted. Based on the position of the samples on the plots the doctors can read/interpret the diagnosis. If one is interested in monitoring and determining the effect of the treatments started in the patients, the script/test can be used for this purpose as well. Here the same procedure is performed as previously mentioned, but the samples are collected after the end of surgery (e.g. 2, 4, and/or 8 hours postoperatively). Depending on the effect of the initiated treatments, a patient's position in the plots will eventually move towards recovery (higher scores levels).

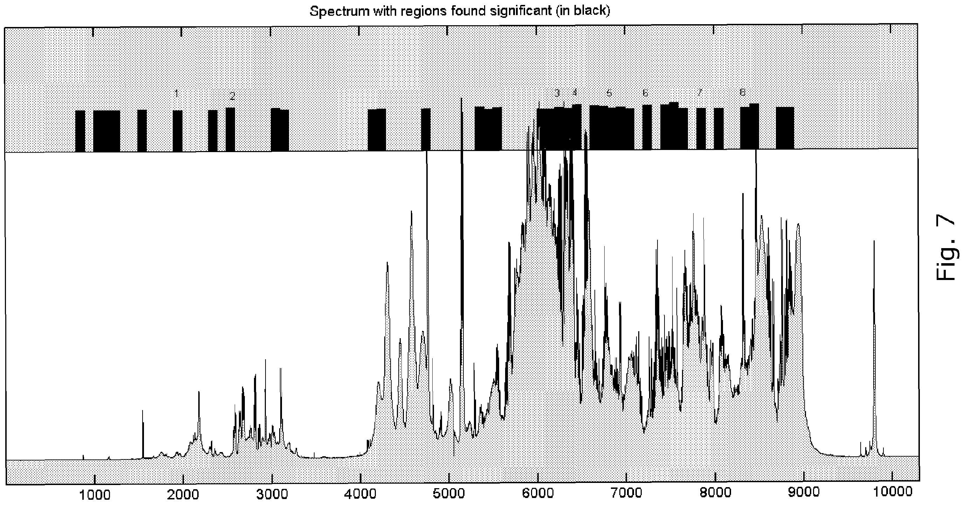

To show the predictive power of our approach, the PLS regression modelling was applied on preprocessed spectral data with the purpose of predicting the diagnostic $PaO_2$ values measured three days postoperatively. Already before patients are coupled to the CPB, specific ROIs on NMR spectra (Figure 7) show 0.70 cross-validated correlation ($R^2$ = 0.70) with the $PaO_2$ values measured 3 days postoperatively, with a root mean-square error of cross-validation (RMSECV) of 0.75 kPa (5.62 mmHg) (Table 5). After weaning from CPB, a 0.91 correlation and a 0.40 kPa (2.62 mmHg) prediction error can be detected by NMR (Table 5). Models conducted on samples collected 2 hours, 4 hours and 8 hours postoperatively have $R^2$ > 0.93 and RMSECV < 0.36kPa (Table 5).

**Table 5:.** For the validation purpose the Venetian-Blinds cross-validation with 10 segments was applied. Here 10 consecutive samples (5 patients in duplicate) were removed and a model was created. Following, the 10 samples were predicted. This procedure was repeated until all samples were removed once. Due to the limitation of not having completely new samples to test the models, the values achieved in this work are relative and represent the average results for removing and predicting 10 of the 100 samples. Already after incision/thoracotomy, our approach can predict the diagnostic outcomes. The cross-validated correlation between the metabolome before CPB and the measured $PaO_2$ on day 3 is 0.7. Increased correlation is observed when the metabolome is measured just after weaning from CPB (0 hours), 2 hours, 4 hours, and 8 hours postoperatively.

| | $R^2$ | | RMSE ($PaO_2$ [kPa]) | | Slope Bias | |
|---|---|---|---|---|---|---|
| | Cal | **CV** | Cal | **CV** | Cal | **CV** |
| After thoracotomy, before CPB | 0.94 | **0.70** | 0.332 | **0.749** | 0 | **0.021** |
| 0 hours (just after CPB) | 0.98 | **0.91** | 0.146 | **0.402** | $-3.5*10^{-15}$ | **0.009** |
| 2 hours (post-CPB) | 0.99 | **0.93** | 0.131 | **0.362** | $-2.6*10^{-15}$ | **-0.001** |
| 4 hours (post-CPB) | 0.99 | **0.95** | 0.123 | **0.302** | $-2.6*10^{-15}$ | **-0.011** |
| 8 hours (post-CPB) | 0.99 | **0.94** | 0.127 | **0.340** | $-1.7*10^{-15}$ | **-0.013** |

The binary classifier PLS-DA was performed to detect the sensitivity and specificity of our method in diagnosing hypoxemia and in determining the risk of ARDS while patients were still undergoing the surgery. After thoracotomy, NMR with machine learning show a 77.8% CV sensitivity and 84.2% CV specificity towards differentiating between hypoxemic and unaffected patients (Table 6). Waiting until weaning from CPB, resulted in a significant increase in the method's accuracy, with 88.2% CV sensitivity and 92.2% CV specificity (Table 6). Also, applying the modeling on samples collected 2 hours, 4 hours and 8 hours postoperatively shows good agreement with aforementioned results (Table 6), indicating the great value of our approach in both diagnosis and monitoring of initiated therapeutical treatments.

**Table 6:** PLSDA applied on the selected ROIs and the diagnosis labels (hypoxemia/none) determine the diagnostic outcome while patients are still undergoing surgery. The 8 hours following surgery still reveal good sensitivity and specificity, indicating their value in diagnosis and monitoring of new treatment options. The results are shown for both the calibrated (Cal) and Venetian-Blinds cross-validated (CV) data.

| Time point | Sensitivity | | Specificity | | Class error | | RMSE | | 500x Permutation testing (p-val) |
|---|---|---|---|---|---|---|---|---|---|
| | Cal | **CV** | Cal | **CV** | Cal | **CV** | Cal | **CV** | CV |
| Pre-CPB | 0.833 | **0.778** | 0.952 | **0.842** | 0.107 | **0.190** | 0.292 | **0.392** | <0.001 |
| 0 h | 1.00 | **0.882** | 0.984 | **0.922** | 0.008 | **0.098** | 0.184 | **0.337** | <0.001 |
| 2 h | 1.00 | **0.912** | 0.984 | **0.938** | 0.007 | **0.075** | 0.179 | **0.075** | <0.001 |
| 4 h | 0.971 | **0.882** | 0.917 | **0.883** | 0.056 | **0.117** | 0.321 | **0.405** | <0.001 |
| 8 h | 0.971 | **0.943** | 0.967 | **0.917** | 0.031 | **0.070** | 0.226 | **0.362** | <0.001 |

*Conclusion*

The present example provides a method for screening and early diagnosis of hypoxemia during cardiac surgery, allowing for direct support of surgeons in the operation theatre. Because the method may lead to diagnostic results within 15-20 minutes, the doctors can make critical and vital decisions during surgery. Also, because the method may provide up to 500 spectra references run on serum collected at different time points, it also allows doctors and nurses to monitor the effect of treatments in process several hours postoperatively.

Newly diagnosed patient samples can also be added to the provided database together with the known outcome, thus leading to a continuously growing and improving database for more accurate prediction.

**Example 6**

*Aim*

Test if Inosine; Dopamine; Dihydroxyphenylalanine (Dopa); Hypoxanthine; and Trigonelline are biomarkers in patients with an increased risk of developing hypoxemia after having had open cardiac surgery with the use of cardiopulmonary bypass (CPB).

*Materials and methods*

*Patient group and clinical outcome*

Serum samples were obtained from fifty (n=50) patients undergoing coronary artery bypass grafting (CABG). Three days postoperatively, 18 patients showed no signs of hypoxemia; while 32 developed hypoxemia with $PaO_2$ below normal values, of which 9 developed severe hypoxemia.

*Samples preparation*

To avoid preanalytical bias due to sample collection, all blood samples were collected and prepared by the same person. Blood samples were obtained from both the left atrium (LA) and pulmonary artery (PA) after sternotomy but before the attachment to the CPB, precisely after weaning from the CPB circuit (0 hours), and 2, 4, 8, and 20 hours ater weaning from the CPB circuit. To obtain serum samples, blood was allowed to clot at room temperature for 30 minutes, and was subsequently centrifuged at 3000 rpm for 10 minutes. Aliquots of LA and PA serum were immediately stored at -80 °C until assayed.

*Sample preparation for NMR*

Prior to NMR measurements, samples were thawed for 2h at 4°C, vortexed, and centrifuged for 5 minutes at 4°C and 14000 rpm to remove cells and other precipitated materials. Aliquots of 400 $\mu$L of supernatant were mixed with 200 $\mu$L 0.2 M phosphate buffer (pH 7.4, uncorrected meter reading) in $^2H_2O$ (99% $^2H$) to minimize variations in pH and to reduce serum viscosity. Throughout the whole process, the samples were kept on ice. The mixture was pipetted into a 5-mm NMR tube and NMR analysis was performed.

*NMR Measurements*

Spectra were acquired on a Bruker DRX-600 NMR spectrometer (Bruker BioSpin, Germany and Switzerland) equipped with a TXI (hydrogen, carbon, nitrogen) probe (Bruker BioSpin, Switzerland) operating at 600.13 MHz for $^1H$. The experiments were acquired at a constant temperature of 310.1 K (37 °C). For the analysis, a $T_2$ relaxation-edited Carr-Purcell-Meiboom-Gill (CPMG) experiment was used. This experiment attenuates broad signals from slowly tumbling proteins and lipoproteins. 128 free induction decays (FIDs) were collected with 32768 complex data points over a spectral width of 11.97 ppm and an acquisition time of 2.28s. A relaxation delay of 2s was applied between each FID during which weak continuous wave irradiation (yBi/2n = 26.6 Hz) was applied at the water frequency (presaturation). The total spin-echo relaxation delay was 67.4 ms and consisted of ($\tau$-$\pi$-$\tau$) elements, where $\tau$ is a delay of 0.4ms and $n$ is a 180° pulse of 22$\mu$s length. Spectral processing was carried out in TopSpin version 2.1 (Bruker BioSpin, Germany). Prior to Fourier transformation, exponential multiplication corresponding to a line broadening of 0.3 Hz was applied to the FIDs, which were further zero-filled by a factor of 2 to double the number of points. Spectra were manually phase- and baseline-corrected, and the methyl signal of alanine was used as chemical shift reference at 1.48 ppm.

For metabolite identification, three types of two-dimensional (2D) experiments were acquired. 2D J-resolved $^1H$-NMR experiments, with water pre-saturation during a 2s relaxation delay were acquired with 8 FIDs for each of the 80 increments. Spectral width was set to 11.6 ppm and 54.7 Hz in F2 and F1 direction, respectively. Prior to 2D-Fourier transformation, both dimensions were multiplied by an unshifted sine bell function and the number of points was doubled by zero-filling. Further, spectra were tilted by 45° and symmetrized along the F1 axis. After processing, the skyline projections of 2D J-resolved spectra were manually baseline corrected and calibrated using the center of the methyl signal of lactate. 2D homonuclear $^1$H,$^1$H-TOCSY (Total Correlation Spectroscopy) and $^1$H,$^{13}$C-HSQC (Heteronuclear Single Quantum Coherence) spectra with presaturation were run on representative samples, with different number of FIDs, increments, spectral widths and mixing times in order to focus on different spectral regions. Information obtained from these spectra was used to find matching metabolites in the Human Metabolome Database. Further metabolite assignments were performed using AMIX (v. 3.9.10, Bruker BioSpin), BRUKER bbiorefcode databases (2.7.0-2.7.3), and literature.

*Quantification of NMR data*

This was performed using the AMIX multi integration tool. Peaks of interest were integrated using the line shape analysis option with a fixed noise factor of 3.5 and a line shape threshold of 0.01. Peaks were integrated using the sum of all points in the region as the integration mode, and these integrals were used as relative concentrations as arbitrary units (a.u.).

*NMR data preprocessing*

CPMG spectra were converted into an n-by-m matrix (n= 590 LA and PA samples from 50 patients, m= 8700 equal buckets of 0.001 ppm width) in AMIX (Analysis of MIXtures software package, version 3.9.10, Bruker BioSpin, Germany) using the region between $\delta$ 9 to 0 ppm, and excluding the water signal region between 4.80-4.5 ppm. Further processing and multivariate modelling were carried out in MATLAB R2011b[56] coupled with PLS-Toolbox 6.5 (Eigenvector Research, Wenatchee, WA). Binned data was *i*Coshifted[57], normalized to total intensity, log transformed, and mean centered.

*Multivariate modelling*

Partial least-squares discriminant analysis (PLS-DA) was applied to identify variables important in sample discrimination and the most significant variables obtained by the method were identified.

*Statistics*

The Statistical Package and Services Solutions (SPSS) software v23.0 was used for the statistical analysis. 2-way ANOVA with Tukey HSD multiple comparison post-hoc tests was used to analyse differences within and between groups. The groups were composed by the time points before CPB (-CPB), 0 hours after CPB (+CPB), 2, 4, 8, and 20 hours after CPB, and the classes unaffected ('none'), mildly affected by hypoxemia ('mild'), and severely affected by hypoxemia ('severe'). Significance was set at $p < 0.05$.
In order to visualize changes between the time points and the groups, the means and standard deviation with the corresponding 95% confidence intervals (CIs) were calculated for each of the significant metabolites (e.g. Trigonelline, Hypoxanthine, Dopamine, Dihydroxyphenylalanine (Dopa) and Inosine).

Results:

Inosine, Dopamine, Dihydroxyphenylalanine (Dopa), Hypoxanthine, and Trigonelline measured after sternotomy but before CPB, after weaning from the CPB (0h), and 2, 4, 8, and 20 h postoperatively were found significantly changing with time and with the risk of developing postoperative hypoxemia (Figure 8A-E/Table 7) at an early stage after weaning from the CPB. The metabolites show consistent trends from none-to-mild-to-severe acute lung injury (Figure 8A-E), indicating their correlation to the degree of later pulmonary dysfunction and their possible function as predictive biomarkers. The metabolic regulation is shown in Figure 8A-E and explained in Table 7.
This is the first study presenting predictive biomarkers of postoperative acute lung injury just after weaning from the CPB or 72h before hypoxemia was diagnosed. The list of the relevant biomarkers found being significantly different within the groups is listed in the tables below.

**Table 7.** Metabolites found significantly changing with time according to the later diagnosis. The timepoins were consisting of samples collected after sternotomy but before CPB, right after weaning from CPB (0h), 2h, 4h, 8h, and 20h after CPB; while the class labels were consisting of unaffected patients ('none'), mildly affected by hypoxemia ('mild'), and severely affected by hypoxemia ('severe')

| 2-way ANOVA | Time Sig. p-value | Class Sig. p-value | Regulation considered indication of the risk of developing hypoxemia |
|---|---|---|---|
| Trigonelline | 1.7E-05 | 1.8E-05 | Lower levels in hypoxemia |
| Hypoxanthine | 1.5E-11 | 2.7E-04 | Lower levels in hypoxemia |
| Inosine | 8.6E-25 | 0.003 | Lower levels in hypoxemia |
| Dopamine | 2.6E-87 | 0.05 | Lower levels in hypoxemia |
| Dihydroxyphenylalanine (Dopa) | 3.9E-14 | 2.04E-09 | Higher levels in hypoxemia |

*Conclusion*

Preliminary data indicates that Inosine; Dopamine; Hypoxanthine; and Trigonelline are down-regulated while Dihydroxyphenylalanine (Dopa) are up-regulated in patients at risk of developing hypoxemia after having had open cardiac surgery with the use of cardiopulmonary bypass (CPB) at an early stage after end surgery.

**DISCUSSION OF RESULTS**

It has been investigated whether the metabolome in patients progressing into postoperative hypoxemia, changes so early and dramatically that this complication could be predicted at least 2-3 days before the clinical signs. NMR spectra (Figure 1) and multivariate modelling accurately related perturbations in the metabolome recorded during and after weaning from CPB with later diagnostic scores. Considering the challenges involved in predicting acute lung injury due to the lack of specific biomarkers, the presented method may therefore enable early disease detection.

From serum samples collected first morning postoperatively, several metabolites were identified varying between patients (Figures 3, 4, and 8). Of these, several markers significantly correlated with $PaO_2$ ($p \leq 0.05$) and showed outstanding diagnostic power, with AUCs>0.8 (see tables above). These are markers of antioxidant status and peroxidation (histidine, PUFA); inducible nitric oxide (iNOS) production (arginine); ATP depletion and reactive oxygen species (ROS) formation (citrate); inflammation (arachidonic and eicosapentanoic acid, membrane injury (phospholipids); and mitochondrial dysfunction (carnitine). The AUC and detection rates for acute lung injury seen in this study are the first of their kind; no other similar studies have reported the use of metabonomics in predicting postoperative outcomes.

This suggests suggest an impaired oxidative phosphorylation. Carnitine, normally found in mitochondria and required for transport of FA across the membrane for β-oxidation, was significantly increased in patients with hypoxemia, which may also indicate impaired oxidative phosphorylation and a possible leakage into the bloodstream. Supporting this, isobutyrylglycine, a marker of impaired mitochondrial FA β-oxidation, was also associated with severity of hypoxemia. Mitochondrial impairments enhance oxidative stress, causing alveolar cell death. Under homeostatic conditions, however, various antioxidants are capable of alleviating the damaging effects of ROS from the system, but an insufficient antioxidant barrier cannot counteract this damage.

Membrane bound phospholipids and PUFA are susceptible to peroxidation leading to the formation of highly reactive hydroperoxides. These react with many biochemical substances having enormous impacts on normal cellular functioning, including endothelial activation and surfactant phospholipid disruption. PUFA and adipic acid (a byproduct of peroxidation) levels correlated with later pulmonary dysfunction, whereas plasmalogen (implicated in protection against ROS and peroxinitrite formation) levels were decreased, especially in the severe hypoxemic group.

Choline, phospholipids, 1,2-DAG, PUFA, and cholesterol are essential for structural integrity and cell membrane signalling. An increase in their levels reflects an activation of the protective mechanism and a possible structural derangement in patients progressing into disease. Lipids are highly interconnected signalling molecules that regulate metabolic, innate immune and inflammatory processes, and alteration in one lipid will automatically trigger major deregulation in several signalling pathways causing profound physiological responses. The levels of arachidonic and eicosapentanoic acids were higher in patients with acute lung injury, indicating an increased inflammatory environment. These PUFA may be released by activation of phospholipase $A_2$, hydrolyzing membrane glycerophospholipids, which may also indicate cell membrane detachment. This is known to be one of the characteristics of acute lung injury. Because one of the properties of this disease is dysfunction of alveolar surfactants, the increased levels of some phospholipids, in samples of hypoxemic patients, may also be partly explained by surfactant leakage, which could indicate a damaged endothelial-alveolar barrier.

Decreased arginine levels were observed in the group developing severe hypoxemia, Blood arginine depletion has previously been linked to acute lung injury, sepsis and cystic fibrosis, and supplementation of arginine was observed to reduce inflammation. This deficiency may have triggered production of superoxides, which further interact with NO producing peroxynitrites, ammonia accumulation, and increased iNOS expression, causing pulmonary microvasculature disruption and tissue damage.

N-Acetylated glucosamine was also found positively correlating with disease. This carbohydrate is-part of pentose metabolism, which is known to be more active under stress, especially under hypotonic stress conditions. This is the first study of its kind, looking at metabolic changes on the first postoperative day following CABG in patients at-risk of developing ALI/ARDS due to the use of CPB. As described above, our findings suggest defects in the mitochondrial respiratory system affecting ROS generation, impaired antioxidant state, increased peroxidation and oxidative stress, disruption of unprotected cell membranes, impaired surfactant production, in patients developing hypoxemia. TCA, arginine, and PUFA take part in a wide range of biological reactions participating in both beneficial and detrimental results, thus therapeutic interventions targeting their paths may open new strategies to prevent postoperative acute lung injury.

We have identified metabolite hallmarks of hypoxemia, which may bridge the gap between pathogenesis and full-blown disease. Markers such as arachidonic and eicosapentanoic acid, citrate, carnitine, glycine, phenylalanine, arginine, and histidine serve as central nodes in their metabolism and therefore have a high impact in predicting disease progression. No single metabolite captured the complexity of injury alone, as different pathways simultaneously showed imbalances. Therefore, to prevent early progression into hypoxemia, therapeutic targeting several pathways would be more effective.

## Claims

1. A method for determining the risk of developing hypoxemia, the method comprising

- providing a biological sample from a subject, said subject having had open cardiac surgery with the use of cardiopulmonary bypass (CPB), wherein said sample has been provided from said subject between start of surgery and 72 hours after detachment from the CPB circuit;
- determining the level of at least one biomarker selected from the group consisting of carnitine, isobutyrylglycine, N-Acetyl-Glucosamine, arachidonic and eicosapentanoic acid, isobutyric acid and citrate; and/or
- determining the ratio between levels of biomarkers of at least one ratio selected from the group of ratios selected from citrate/phenylalanine, polyunsaturated fatty acids (PUFA)/phenylalanine, alanine/phenylalanine, citrate/ trimethylamine-N-oxide (TMAO), carnitine/TMAO, isobutyric acid/phenylalanine, and isobutyrylglycine/arginine;
- comparing said levels of one or more biomarkers or ratios to one or more reference levels;
- determining that said subject is at risk of developing hypoxemia if said level of one or more biomarkers are above the one or more reference levels, or determining that said subject is not at risk of developing hypoxemia, if said one or more levels are equal to or below the one or more reference levels; and/or
- determining that said subject is at risk of developing hypoxemia if said one or more ratios between levels of biomarkers are above the one or more reference levels, or determining that said subject is not at risk of developing hypoxemia, if said level of one or more ratios between levels of biomarkers are equal to or below the one or more reference levels.

2. The method according to claim 1, wherein said sample has been provided from said subject within 48 hours after detachment from the CPB circuit, such as within 30 hours, such as within 20 hours, such as within 16 hours, such as within 10 hours, such as within 5 hours, such as within 2 hours, such as within 1 hour after detachment from the CPB circuit.

3. The method according to claim 1, wherein said sample has been provided from said subject before detachment from the CPB circuit until 2 hours after detachment from the CPB circuit.

4. The method according to any of the preceding claims, wherein at least the level of at least two biomarkers and/or at least one ratio between biomarkers are determined.

5. The method according to any of the preceding claims, wherein at least the levels of carnitine and citrate are determined.

6. The method according to any of the preceding claims, wherein the sample is a blood sample, such as whole blood, serum and/or plasma.

7. The method according to any of the previous claims, wherein said level of biomarkers are determined by a method selected from the group consisting of mass spectrometry (GC-MS, LC-MS), HPLC, Raman, NIR, and NMR spectroscopy.

8. The method according to any of the preceding claim, wherein hypoxemia is a $PaO_2/FiO_2$ below 40 kPa (300 mmHg).

9. The method according to any of the preceding claims, wherein said open heart surgery is selected from the group consisting of coronary artery bypass grafting and valve surgery.

10. The method according to any of the preceding claims, comprising applying NMR spectroscopy on said biological samples such as performing a 1D NMR such as CPMG, so as to arrive at a resulting spectrum covering a spectral range representative of said biomarkers, and applying a computer-based algorithm in response, so as to determine an output indicative of said subject being at risk of developing hypoxemia.

11. The method according to claim 10, wherein said biological sample has been provided from said subject between a time during surgery, such as from weaning from the CPB circuit until 8 hours after weaning from the CPB circuit.

12. The method according to claim 10 or 11, wherein a combination of spectral intervals are selected to cover all of: carnitine, isobutyrylglycine, N-Acetyl-Glucosamine, arachidonic and eicosapentanoic acid, isobutyric acid and citrate.

13. The method according to any of claims 10-12, wherein said computer-based statistical analysis involves applying a machine learning algorithm.

14. A method for evaluating the efficacy of treatment to prevent hypoxemia, the method comprising

- completing the method according to any of claims 1-13;
for a subject considered at risk of developing hypoxemia, evaluating whether a treatment protocol for the prevention of hypoxemia, results in that said subject is not developing hypoxemia within one week after weaning from the CPB circuit.

**Patentansprüche**

1. Verfahren zum Bestimmen des Risikos, Hypoxämie zu entwickeln, wobei das Verfahren umfasst:

- Bereitstellen einer biologischen Probe von einer Person, wobei die Person eine offene kardiale Operation unter Verwendung eines kardiopulmonalen Bypasses (CPB) hatte, wobei die Probe von der Person zwischen Beginn der Operation und 72 Stunden nach Ablösen von dem CPB-Kreislauf bereitgestellt wurde;
- Bestimmen des Gehalts von mindestens einem Biomarker, ausgewählt aus der Gruppe bestehend aus Carnitin, Isobutyrylglycin, N-Acetyl-Glucosamin, Arachidon und Eicosapentansäure, Isobuttersäure und Citrat; und/oder
- Bestimmen des Verhältnisses zwischen den Biomarkern von mindestens einem Verhältnis, ausgewählt aus der Gruppe der Verhältnisse, ausgewählt aus Citrat/Phenylalanin, mehrfach ungesättigten Fettsäuren (PUFA)/Phenylalanin, Alanin/Phenylalanin, Citrat/Trimethylamin-N-oxid (TMAO), Carnitin/TMAO, Isobuttersäure/Phenylalanin und Isobutyrylglycin/Arginin;
- Vergleichen der Werte von einem oder mehreren Biomarkern oder Verhältnissen mit einem oder mehreren Referenzgehalten;
- Bestimmen, dass die Person Gefahr läuft, Hypoxämie zu entwickeln, wenn der Gehalt an einem oder mehreren Biomarkern über dem einen oder den mehreren Referenzgehalt liegt, oder Bestimmen, dass die Person nicht Gefahr läuft, Hypoxämie zu entwickeln, wenn der eine oder die mehreren Gehalte gleich oder unter dem einen oder den mehreren Referenzgehalten sind; und/oder

- Bestimmen, dass die Person Gefahr läuft, Hypoxämie zu entwickeln, wenn das eine oder die mehreren Verhältnisse zwischen den Gehalten von Biomarkern über dem einen oder den mehreren Referenzgehalt liegt, oder Bestimmen, dass die Person nicht Gefahr läuft, Hypoxämie zu entwickeln, wenn der Gehalt von einem oder mehreren Verhältnissen zwischen den Gehalten von Biomarkern gleich oder unter dem einen oder den mehreren Referenzgehalten ist.

2. Verfahren nach Anspruch 1, wobei die Probe von der Person innerhalb von 48 Stunden nach dem Ablösen vom CPB-Kreislauf, wie etwa innerhalb von 30 Stunden, wie etwa innerhalb von 20 Stunden, wie etwa innerhalb von 16 Stunden, wie etwa innerhalb von 10 Stunden, wie etwa innerhalb von 5 Stunden, wie etwa innerhalb von 2 Stunden, wie etwa innerhalb von 1 Stunde nach dem Ablösen vom CPB-Kreislauf, bereitgestellt wurde.

3. Verfahren nach Anspruch 1, wobei die Probe von der Person vor dem Ablösen vom CPB-Kreislauf bis 2 Stunden nach dem Ablösen vom CPB-Kreislauf bereitgestellt wurde.

4. Verfahren nach einem der vorstehenden Ansprüche, wobei mindestens der Gehalt von mindestens zwei Biomarkern und/oder mindestens ein Verhältnis zwischen Biomarkern bestimmt wird.

5. Verfahren nach einem der vorstehenden Ansprüche, wobei mindestens die Gehalte an Carnitin und Citrat bestimmt werden.

6. Verfahren nach einem der vorstehenden Ansprüche, wobei die Probe eine Blutprobe ist, wie etwa Vollblut, Serum und/oder Plasma.

7. Verfahren nach einem der vorherigen Ansprüche, wobei der Gehalt an Biomarkern durch ein Verfahren bestimmt wird, das aus der Gruppe ausgewählt ist, die aus Massenspektrometrie (GC-MS, LC-MS), HPLC, Raman, NIR und NMR-Spektroskopie besteht.

8. Verfahren nach einem der vorstehenden Ansprüche, wobei Hypoxämie ein $PaO_2/FiO_2$ unter 40 kPa (300 mmHg) ist.

9. Verfahren nach einem der vorstehenden Ansprüche, wobei die offene Herzoperation ausgewählt ist aus der Gruppe bestehend aus koronarer Bypass-Operation und Klappenoperation.

10. Verfahren nach einem der vorstehenden Ansprüche, umfassend das Anwenden von NMR-Spektroskopie auf die biologischen Proben, wie etwa das Durchführen einer 1D-NMR wie etwa CPMG, um zu einem resultierenden Spektrum zu gelangen, das einen für die Biomarker repräsentativen Spektralbereich abdeckt, und das Anwenden eines computergestützten Algorithmus als Reaktion darauf, um so eine Ausgabe zu bestimmen, die anzeigt, dass die Person Gefahr läuft, Hypoxämie zu entwickeln.

11. Verfahren nach Anspruch 10, wobei die biologische Probe von der Person zwischen einer Zeit während der Operation, wie beispielsweise dem Entwöhnen von dem CPB-Kreislauf, und 8 Stunden nach dem Entwöhnen von dem CPB-Kreislauf bereitgestellt wurde.

12. Verfahren nach Anspruch 10 oder 11, wobei eine Kombination von Spektralintervallen ausgewählt wird, um alle von: Carnitin, Isobutyrylglycin, N-Acetyl-Glucosamin, Arachidon und Eicosapentansäure, Isobuttersäure und Citrat abzudecken.

13. Verfahren nach einem der Ansprüche 10 - 12, wobei die computergestützte statistische Analyse die Anwendung eines maschinellen Lernalgorithmus beinhaltet.

14. Verfahren zum Bewerten der Wirksamkeit einer Behandlung zur Verhinderung von Hypoxämie, wobei das Verfahren umfasst

- Vervollständigen des Verfahrens nach einem der Ansprüche 1-13;
für eine Person, die als gefährdet angesehen wird, Hypoxämie zu entwickeln, Bewerten, ob ein Behandlungsprotokoll zur Verhinderung von Hypoxämie dazu führt, dass die Person nicht innerhalb einer Woche nach dem Entwöhnen von dem CPB-Kreislauf Hypoxämie entwickelt.

**Revendications**

1. Procédé pour déterminer le risque de développer une hypoxémie, ledit procédé comprenant les étapes consistant à :

   - partir d'un échantillon biologique provenant d'un sujet, ledit sujet ayant subi une opération à coeur ouvert avec utilisation d'un pontage cardiopulmonaire (CPB), où ledit échantillon a été prélevé chez ledit sujet entre le début de l'opération et 72 heures après le retrait du circuit de CPB ;
   - déterminer le niveau d'au moins un biomarqueur choisi dans le groupe constitué par la carnitine, l'isobutyrylglycine, la N-Acétyl-Glucosamine, l'acide arachidonique et éicosapentanoïque, l'acide isobutyrique et le citrate ; et/ou
   - déterminer le rapport entre les niveaux de biomarqueurs d'au moins un rapport choisi dans le groupe de rapports sélectionnés parmi les suivants : citrate/phénylalanine, acides gras polyinsaturés (PUFA)/phénylalanine, alanine/phénylalanine, citrate/triméthylamine-N-oxyde (TMAO), carnitine/TMAO, acide isobutyrique/phénylalanine, et isobutyrylglycine/arginine ;
   - comparer lesdits niveaux d'un ou de plusieurs biomarqueurs et rapports à un ou plusieurs niveaux de référence ;
   - déterminer que ledit sujet présente un risque de développer une hypoxémie si ledit niveau d'un ou de plusieurs biomarqueurs sont supérieurs à l'un ou à plusieurs des niveaux de référence, ou déterminer que ledit sujet ne présente pas de risque de développer une hypoxémie si ledit ou lesdits niveau(x) sont inférieurs ou égaux à l'un ou à plusieurs des niveaux de référence ; et/ou
   - déterminer que ledit sujet présente un risque de développer une hypoxémie si ledit ou lesdits rapports entre les niveaux de biomarqueurs sont supérieurs à l'un ou à plusieurs des niveaux de référence, ou déterminer que ledit sujet ne présente pas de risque de développer une hypoxémie si ledit niveau de l'un ou de plusieurs des rapports entre les niveaux de biomarqueurs est inférieur ou égal à l'un ou à plusieurs des niveaux de référence.

2. Procédé selon la revendication 1, dans lequel ledit échantillon a été prélevé chez ledit sujet dans un intervalle de 48 heures après le retrait du circuit de CPB, comme par exemple, dans un intervalle de 30 heures, dans un intervalle de 20 heures, dans un intervalle de 16 heures, dans un intervalle de 10 heures, dans un intervalle de 5 heures, dans un intervalle de 2 heures, dans un intervalle de 1 heure après le retrait du circuit de CPB.

3. Procédé selon la revendication 1, dans lequel ledit échantillon a été prélevé chez ledit sujet avant le retrait du circuit de CPB et jusqu'à 2 heures après le retrait du circuit de CPB.

4. Procédé selon l'une quelconque des revendications précédentes, dans lequel au moins le niveau d'au moins deux biomarqueurs et/ou au moins un rapport entre les biomarqueurs sont déterminés.

5. Procédé selon l'une quelconque des revendications précédentes, dans lequel au moins les niveaux de carnitine et de citrate sont déterminés.

6. Procédé selon l'une quelconque des revendications précédentes, dans lequel l'échantillon est un échantillon de sang, tel que du sang total, du sérum et/ou du plasma.

7. Procédé selon l'une quelconque des revendications précédentes, dans lequel lesdits niveaux de biomarqueurs sont déterminés par un procédé choisi dans le groupe constitué par la spectrométrie de masse (CG-SM, CL-SM), la CLHP, la spectroscopie Raman, NIR et RMN.

8. Procédé selon l'une quelconque des revendications précédentes, dans lequel l'hypoxémie correspond à un $PaO_2/FiO_2$ inférieur à 40 kPa (300 mmHg).

9. Procédé selon l'une quelconque des revendications précédentes, dans lequel ladite opération à coeur ouvert est choisie dans le groupe constitué par le pontage aorto-coronarien et la chirurgie valvulaire.

10. Procédé selon l'une quelconque des revendications précédentes, comprenant l'application d'une spectroscopie RMN sur lesdits échantillons biologiques, comme la mise en oeuvre d'une RMN 1D telle que CPMG, de manière à obtenir un spectre résultant couvrant un domaine spectral représentatif desdits biomarqueurs, et l'application d'un algorithme informatique en réponse, de manière à déterminer un résultat indiquant si ledit sujet présente un risque de développer une hypoxémie.

**11.** Procédé selon la revendication 10, dans lequel ledit échantillon biologique a été prélevé chez ledit sujet entre un moment au cours de l'opération, tel qu'à partir du sevrage du circuit de CPB, jusqu'à 8 heures après le sevrage du circuit de CPB.

**12.** Procédé selon la revendication 10 ou 11, dans lequel une combinaison d'intervalles spectraux est choisie pour couvrir tout à la fois: la carnitine, l'isobutyrylglycine, la L-Acétyl-Glucosamine, l'acide arachidonique et éicosapentanoïque, l'acide isobutyrique et le citrate.

**13.** Procédé selon l'une quelconque des revendications 10-12, dans lequel ladite analyse statistique informatique implique l'application d'un algorithme d'apprentissage automatique.

**14.** Procédé pour évaluer l'efficacité d'un traitement de prévention de l'hypoxémie, ledit procédé comprenant :

- l'achèvement du procédé selon l'une quelconque des revendications 1-13 ;

pour un sujet considéré comme présentant un risque de développer une hypoxémie, l'évaluation pour savoir si un protocole de traitement pour la prévention de l'hypoxémie aboutit au fait que ledit sujet ne développe pas d'hypoxémie dans la semaine qui suit le sevrage du circuit de CPB.

Fig. 1

Fig. 2, continues

Fig. 2, continued

## Fold Change

-65%

0

75%

**** Carnitine
**** Formic acid
*** Ara+Epa
**** NAcGlc
**** Citrate
* Urocanate
*** Phenylalanine
**** Lysine
**** Glycine
* 1-Methylhistidine
* Isobutyric acid
** Plasmalogen
** Lyso-PC
Citrulline
** Arginine
** Glutamate
*** Isobutyrylglycine
*** Free FA + Adipate
** Pyruvate
** UFA
** Malonic acid
* Acetate
** Acetoacetate
** 2-Ketobutyrate
** GPE
* Choline

# Fig. 3, continues

Fig. 3, continued

Fig. 4

Fig. 5

Fig. 6

Fig. 7

Fig. 8, continues

**D**

Error Bars: 95% CI

**E**

Error Bars: 95% CI

Fig. 8, continued

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOJKO et al.** *Journal of Pharmaceutical Analysis,* 01 February 2014, vol. 4 (1), 6-13
- **SERKOVA et al.** *American Journal of respiratory and Critical Care Medicine,* 2011, vol. 184 (6), 647-655
- **STRINGER et al.** *American Journal of Physiology. Lung Cellular and Molecular Physiology,* 01 October 2010, vol. 300 (1), 4-11
- **SHAH et al.** *Journal of Thoracic and Cardiovascular Surgery,* 15 September 2011, vol. 143 (4), 873-878
- **AGOSTONI et al.** *European Respiratory Journal,* 22 July 2010, vol. 37 (4), 841-847
- **ENGELS et al.** *International Journal of Artificial Organs,* 01 September 2014, vol. 37 (9), 679-687
- **ENGELS et al.** *Journal of Cardiothoracic Surgery,* 09 January 2013, vol. 8, 4
- **ENGELS et al.** *Disease Markers,* 01 January 2015, vol. 18 (3), 203-210